# EUROPÄISCHE PATENTSCHRIFT

(11) **EP 2 748 310 B1**
(45) Veröffentlichungstag und Bekanntmachung des Hinweises auf die Patenterteilung: **07.10.2015**
(21) Anmeldenummer: 12761701.7
(22) Anmeldetag: 09.08.2012
(51) Int. Cl.: C12N 9/00, C12P 7/24

(54) **ENZYMATISCHE ALKENSPALTUNG**
ENZYMATIC ALKENE CLEAVAGE
DISSOCIATION ENZYMATIQUE D'ALCÈNE

(30) Priorität: 26.08.2011 AT 12212011
(43) Veröffentlichungstag der Anmeldung: 02.07.2014
(73) Patentinhaber: Universität Graz, 8010 Graz (AT)
(72) Erfinder: RAJAGOPALAN, Aashrita, Chennai-600041 (IN); KROUTIL, Wolfgang, 8042 Graz (AT); SCHOBER, Markus, 9433 St. Andrae (AT)
(74) Vertreter: Ellmeyer, Wolfgang
(86) Internationale Anmeldenummer: PCT/AT2012/050113
(87) Internationale Veröffentlichungsnummer: WO 2013/029076

(56) Entgegenhaltungen:
- Aashrita Rajagopalan ET AL: "Biocatalytic C=C double bond cleavage by an enzyme from Trametes hirsuta: Unexpected metal dependence", , 1. November 2011 (2011-11-01), XP055048948, Gefunden im Internet: URL:http://www.biotrains.eu/documents/Post er-BIOTRANS-italy-october 2011-biotrains website.pdf [gefunden am 2013-01-09]
- MIGUEL LARA ET AL: "Biocatalytic Cleavage of Alkenes with O 2 and Trametes hirsuta G FCC 047", EUROPEAN JOURNAL OF ORGANIC CHEMISTRY, Bd. 2008, Nr. 21, 1. Juli 2008 (2008-07-01), Seiten 3668-3672, XP055049039, ISSN: 1434-193X, DOI: 10.1002/ejoc.200800261
- KURLEMANN N ET AL: "Asymmetric synthesis of chiral 2-hydroxy ketones by coupled biocatalytic alkene oxidation and C?C bond formation", JOURNAL OF MOLECULAR CATALYSIS. B, ENZYMATIC, ELSEVIER, AMSTERDAM, NL, Bd. 61, Nr. 1-2, 1. November 2009 (2009-11-01), Seiten 111-116, XP026670691, ISSN: 1381-1177, DOI: 10.1016/J.MOLCATB.2008.08.009 [gefunden am 2008-08-26]
- MANG ET AL: "Optimization of a biocatalytic single-step alkene cleavage of aryl alkenes", TETRAHEDRON, ELSEVIER SCIENCE PUBLISHERS, AMSTERDAM, NL, Bd. 63, Nr. 16, 15. März 2007 (2007-03-15) , Seiten 3350-3354, XP005924227, ISSN: 0040-4020, DOI: 10.1016/J.TET.2007.02.034
- DATABASE UniProt [Online] 26. Februar 2008 (2008-02-26), "SubName: Full=Aspartic peptidase A1;", XP002689904, gefunden im EBI accession no. UNIPROT:B0CYE2 Database accession no. B0CYE2

## Beschreibung

Die Erfindung betrifft ein erstmalig aus einer Pilzkultur isoliertes Enzym, dessen Verwendung bei der Katalyse von Alkenspaltungsreaktionen sowie ein Verfahren zu seiner Herstellung.

### STAND DER TECHNIK

Die Spaltung der aliphatischen Doppelbindung von Vinylaromaten mittels enzymatischer Katalyse ist seit längerem bekannt. Beispielsweise werden in WO 96/22381 A1 und der entsprechenden US 5.861.286 A solche Oxidationen in Gegenwart von Proteinen, die mitunter diverse Metalloproteine oder Enzyme, z.B. Hämoglobin oder Protoporphyrin, sein können, und von Metallionen beschrieben. Als einziges Beispiel, in dem kein Protein mit Protoporphyrin-Struktur verwendet wird, kommt stattdessen ein Auberginenbrei als Katalysator zum Einsatz, von dem zwar erwähnt wird, er weise aufgrund seines Proteingehalts "eine bestimmte oxidative enzymatische Wirkung" auf, die allerdings nicht näher spezifiziert wird. Field et al., Eur. J. Biochem. 265, 1008-1014 (1999), beschreiben die Oxidation von Isoeugenolacetat mit Lignin-Peroxidase unter Verwendung von H₂O₂, in Gegenwart von Veratrylalkohol. Tadao et al., Bioorg: Med. Chem. Lett. 12(8), 1139-1142 (2002), beschreiben die Spaltung von Stilbenen mittels Lignostilben-α,β-Dioxygenase unter Verwendung von Sauerstoff.

Auch die Erfinder des vorliegenden Anmeldungsgegenstands haben im Zuge früherer Forschungen bereits neue und effiziente enzymkatalysierte Verfahren zur Spaltung von Vinylaromaten entwickelt. So offenbaren sie etwa in der WO 2009/006662 A2 ein Verfahren mittels enzymatischer Katalyse durch bestimmte Peroxidasen und Laccasen, die überraschenderweise Sauerstoff als Substrat nutzen, und in der WO 2010/003161 A1 ein ähnliches Verfahren mittels enzymatischer Katalyse durch bestimmte Hämine ebenfalls in Gegenwart von Sauerstoff.

Außerdem hatten sie schon zuvor festgestellt, dass der Zusatz von Zellen oder Zellextrakten eines bestimmten Pilzes, nämlich von *Trametes hirsuta* (striegelige Tramete) derartige Oxidationen in Gegenwart von Sauerstoff ebenfalls katalysieren kann (Kroutil et al., Angew. Chem. 118, 5325-5328 (2006) und Angew. Chem. Int. Ed. 45, 5201-5203 (2006)), es konnte jedoch nicht geklärt werden, welche(s) Enzym(e) dafür verantwortlich war(en), da die Isolierung der entsprechenden Enzymaktivität bisher stets misstang.

Miguel Lara (EUROPEAN JOURNAL OF ORGANIC CHEMISTRY, Bd. 2008, Nr. 21, 1. Juli 2008, Seiten 3668-3672) offenbart Alken-Spaltung unter Verwendung eines zellfreien Extrakts von Trametes hirsuta.

Ziel der Erfindung war daher die Isolierung und Analyse des Enzyms oder der Enzyme, das/die diese Enzymaktivität aufweist/aufweisen.

### OFFENBARUNG DER ERFINDUNG

Dieses Ziel erreicht die vorliegende Erfindung durch Bereitstellung eines erstmalig isolierten, bislang unbekannten Enzyms, das die Sequenz von Seq.-ID Nr. 1 oder Seq.-ID Nr. 2 aufweist oder umfasst. Dabei entspricht Seq.-ID Nr. 1 der durch Primer-Walking ermittelten Sequenz, während Seq.-ID Nr. 2 die durch abschließende Sequenzierung des dann aus *Trametes hirsuta* isolierten Enzyms erhaltene Sequenz darstellt, die sich lediglich in einer einzigen Aminosäure unterscheiden. Dieser Unterschied betrifft ein Alanin in der abschließenden Sequenz gegenüber Valin in der durch Primer-Walking ermittelten. Da beide Aminosäuren zu den apolaren aliphatischen Aminosäuren zählen, wird eine solche Ersetzung als "konservative Substitution" angesehen, weswegen ein Enzym mit der durch Primer-Walking ermittelten Sequenz mit hoher Wahrscheinlichkeit ähnliche Eigenschaften und Aktivitäten wie die isolierte Aminosäuresequenz aufweisen wird.

Wie Fachleuten auf dem Gebiet der Erfindung bekannt ist, sind die Eigenschaften von Proteinhomologen umso ähnlicher, je stärker die Identität der Aminosäuresequenzen ist. Je höher daher der Grad der Identität der Aminosäuresequenz eines Proteins oder Enzyms mit jener von Seq.-ID Nr. 1 oder Seq.-ID Nr. 2, desto besser wird seine Aktivität als Reaktionskatalysator in den Alkenspaltungsreaktionen sein. Folglich wird ein Protein mit einer Aminosäuresequenz, die zumindest 80 %, vorzugsweise zumindest 90 %, noch bevorzugter zumindest 95 %, insbesondere zumindest 99 %, Identität mit Seq.-ID Nr. 1 oder 2 aufweist, zumindest vergleichbare bis hin zu derselben Aktivität wie das isolierte Enzym aufweisen.

Das isolierte Enzym mit Seq.-ID Nr. 2 zeigte allerdings anfgangs kaum Wirksamkeit als Katalysator für die Spaltung von Alkenen mit Sauerstoff. Überraschenderweise haben die Erfinder jedoch herausgefunden, dass die katalytische Aktivität des Enzyms dramatisch erhöht werden kann, wenn im Reaktionsgemisch Mn³⁺-Ionen anwesend sind. Somit scheint dieses Enzym die Gegenwart von Mangan(III) als Cofaktor zu erfordern, um bei der obigen Reaktion in zufriedenstellendem Ausmaß katalytisch wirksam sein zu können, obwohl Enzyme mit vergleichbaren Aktivitäten im Allgemeinen üblicherweise Eisen- oder Kupferabhängigkeiten besitzen. Zudem sind Enzyme mit ähnlichen Aminosäuresequenzen wie das Enzym der Erfindung üblicherweise Proteinasen, die keinerlei Aktivität als Katalysator in Alkenspaltungsreaktionen zeigen. Aus diesem Grund war die von den Erfindern festgestellte Aktivität umso überraschender.

In einem zweiten Aspekt umfasst die Erfindung die Verwendung eines Enzyms gemäß dem ersten Aspekt der Erfindung - oder eines Proteins mit zumindest 80 %, vorzugsweise zumindest 90 %, noch bevorzugter zumindest 95 %, insbesondere zumindest 99 %, Identität mit Seq.-ID Nr. 1 oder 2 - als Katalysator bei der Spaltung von Vinylaromaten mit Sauerstoff, wobei die Spaltung vorzugsweise in Gegenwart von Mn³⁺-Ionen erfolgt, um die Enzymaktivität zu erhöhen, wie oben ausgeführt wird.

In einem dritten Aspekt umfasst die Erfindung ein Verfahren zur Herstellung eines erfindungsgemäßen Enzyms, indem eine Kultur von *Trametes hirsuta* kultiviert wird und das Enzym aus einem zellfreien Extrakt der Kultur gewonnen wird, wobei vorzugsweise eine Kultur von *Trametes hirsuta* G FCC 047 kultiviert wird, da die Erfinder mit diesem Stamm gute Ergebnisse erzielt haben. Weiters wird die Gewinnung aus dem zellfreien Extrakt in bevorzugten Ausführungsformen mittels einer Kombination aus hydrophober Wechselwirkungschromatographie und Anionenaustauschchromatographie durchgeführt, insbesondere durch hydrophobe Wechselwirkungschromatographie, Anionenaustauschchromatographie und erneut hydrophobe Wechselwirkungschromatographie in dieser Reihenfolge.

In einem vierten Aspekt betrifft die Erfindung schließlich ein Verfahren zur rekombinanten Produktion des Enzyms gemäß dem ersten Aspekt der Erfindung unter Verwendung von für das Enzym kodierender Nucleinsäure, d.h. Nucleinsäure mit einer Nucleotidsequenz gemäß Seq.-ID Nr. 3 oder 4, wobei erstere der für Seq.-ID Nr. 1 kodierenden Nucleotidsequenz und zweitere der für Seq.-ID Nr. 2 kodierenden Nucleotidsequenz entspricht. Die beiden Sequenzen wurden erhalten, wie dies nachstehend ausführlich beschrieben wird. Die für das Enzym kodierende Nucleinsäure wird dabei vorzugsweise in einen Vektor, wie z.B. einen Plasmidvektor, ligiert, mit dem Zellen transformiert werden, die zur Expression des Enzyms in der Lage sind, wie z.B. E. *coli-*Zellen, aber auch andere prokaryotische oder eukaryotische Zellen, wie z.B. *Pichia pastoris.* Nach geeigneter Inkubation der transformierten Zellen wird das Enzym der Erfindung durch fachbekannte Techniken, vorzugsweise als zellfreier Extrakt, aus der Kulturbrühe gewonnen und, vorzugsweise in lyophilisiertem Zustand, gelagert.

Beispiele für die erfindungsgemäßen Verfahren werden später detailliert beschrieben.

### KURZBESCHREIBUNG DER ZEICHNUNGEN

Nachstehend wird die Erfindung unter Bezugnahme auf die beiliegenden Zeichnungen näher beschrieben, die Folgendes darstellen.
Fig. 1 zeigt das Reaktionsschema der Spaltung von *trans*-Anethol mit einem zellfreien Extrakt von *Trametes hirsuta* als Katalysator.
Fig. 2 zeigt das Chromatogramm der hydrophoben Wechselwirkungschromatographie, die als erster Reinigungsschritt durchgeführt wurde.
Fig. 3 zeigt das Chromatogramm der Anionenaustauschchromatographie, die als zweiter Reinigungsschritt durchgeführt wurde.
Fig. 4 zeigt das Elektropherogramm einer SDS-PAGE von Anionenaustauschchromatographie-Proben.
Fig. 5 zeigt das Chromatogramm der hydrophoben Wechselwirkungschromatographie, die als dritter Reinigungsschritt durchgeführt wurde.
Fig. 6 ist eine Ausschnittsvergrößerung des Chromatogramms aus Fig. 5.
Fig. 7 zeigt das Elektropherogramm einer SDS-PAGE der hydrophoben Wechselwirkungschromatographie-Proben aus dem dritten Reinigungsschritt.
Fig. 8 zeigt das Elektropherogramm einer SDS-PAGE der aktivsten Fraktion nach der hydrophoben Wechselwirkungschromatographie.
Fig. 9 zeigt ein Elektropherogramm von PCR-Produkten auf Agarosegel.
Fig. 10 zeigt ein Elektropherogramm der in einen Kloniervektor insertierten PCR-Produkte.
Fig. 11 zeigt ein Fließbild der cDNA-Konstruktion bei der De-Novo-Sequerizierung.
Fig. 12 zeigt ein Elektropherogramm der PCR-Produkte bei Primer-Walking 1.
Fig. 13 zeigt ein Elektropherogramm der PCR-Produkte bei Primer-Walking 2.
Fig. 14 zeigt ClustaW-Sequenzabgleichsergebnisse bei Primer-Walking 3.
Fig. 15 zeigt ein Elektropherogramm der Produkte von optimierten PCR-Reaktionen bei Primer-Walking 3.
Fig. 16 zeigt ein Elektropherogramm der Produkte von Nested-Primer-PCR-Reaktionen bei Primer-Walking 3.
Fig. 17 zeigt die allgemeine Vorgangsweise beim Primer-Walking 4.
Fig. 18 zeigt ein Elektropherogramm der Produkte der dritten PCR-Reaktion bei Primer-Walking 4.
Fig. 19 zeigt ein Elektropherogramm des vollständigen, für das Enzym kodierenden Gens.
Fig. 20 zeigt einen Sequenzabgleich zwischen dem isolierten und dem aus Primer-Walking-Teilstücken zusammengesetzten vollständigen Gen mittels der Clustal 2.1-Software.
Fig. 21 zeigt einen Vergleich der Aminosäuresequenzen, für die das isolierte bzw.
das aus Primer-Walking-Teilstücken zusammengesetzte vollständige Gen kodieren.
Fig. 22 zeigt einen Vergleich der Sequenzen des aus genomischer DNA und des aus cDNA als Templat erhaltenen Gens.
Fig. 23 zeigt ein Elektropherogramm dreier Reaktionsgemische zum Restriktionsverdau des Gens aus dem pJET1.2-Vektor.

### Nomenklatur

Die Bezeichnungen von Nucleotidsequenzen erfolgt hierin unter Verwendung des üblichen Einbuchstabencodes, d.h. A für Adenin, C für Cytosin, G für Guanin und T für Thymin (in Groß- oder Kleinschreibung). Die Bezeichnungen der Aminosäuren der in der vorliegenden Beschreibung und im Sequenzprotokoll offenbarten Aminosäuresequenzen erfolgt im Ein- bzw. Drei-Buchstaben-Code, die nachstehend der Übersicht halber angegeben sind.

| **Aminosäure** | **Dreibuchstabencode** | **Einbuchstabencode** |
|---|---|---|
| Alanin | Ala | A |
| Arginin | Arg | R |
| Asparagin | Asn | N |
| Asparaginsäure | Asp | D |
| Cystein | Cys | C |
| Glutamin | Gln | Q |
| Glutaminsäure | Glu | E |
| Glycin | Gly | G |
| Histidin | His | H |
| Isoleucin | Ile | I |
| Leucin | Leu | L |
| Lysin | Lys | K |
| Methionin | Met | M |
| Phenylalanin | Phe | F |
| Prolin | Pro | P |
| Serin | Ser | S |
| Threonin | Thr | T |
| Tryptophan | Trp | W |
| Tyrosin | Tyr | Y |
| Valin | Val | V |

### DETAILLIERTE BESCHREIBUNG DER ERFINDUNG

### 1.1 Einführung

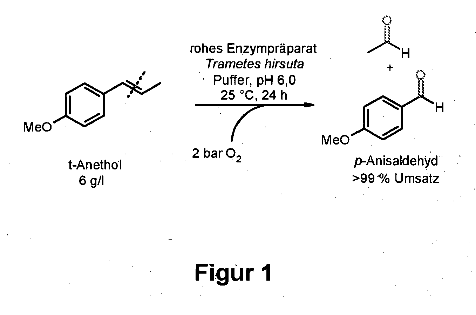

*Trametes hirsuta (Coriolus hirsuta)* ist ein Pilz, der zur Familie der Weißfäulepilze gehört. Alkene, die eine C=C-Doppelbindung benachbart zu einem aromatischen Ring umfassen, wurden gespalten, um die entsprechende Carbonylverbindung zu erhalten, wobei molekularer Sauerstoff als einziges Oxidationsmittel und ein zellfreier Extrakt einer Kultur von *Trametes hirsuta* G FCC 047 als Katalysator verwendet wurden. *trans*-Anethol erwies sich als bestes Substrat für die biokatalytische Alkenspaltung, die *p*-Anisaldehyd als einziges Produkt ergab. Die zur Ausführung dieser Reaktion erforderlichen Bedingungen wurden vorher optimiert und sind in Fig. 1 angeführt.

Die Reinigung dieses alkenspaltenden Enzyms aus dem zellfreien Extrakt einer *Trametes-hirsuta-*Kultur wurde bereits zuvor einige Male versucht. Dies erwies sich jedoch immer als problematisch, da die Aktivität des Enzyms nach zwei oder sogar schon nach einem Reinigungsschritt verloren ging (hydrophobe Wechselwirkungschromatographie, gefolgt von Größenausschlusschromatographie). Die genaue Ursache für diesen Aktivitätsverlust war unbekannt. Es wurde jedoch vermutet, dass während der Säulenreinigung der Enzym-Cofaktor verloren ging, der für die Reaktion verantwortlich ist. Weitere Forschungen wurden durchgeführt, um die Abhängigkeit des Enzyms von Metallen zu überprüfen.

### 1.2 Ergebnisse und Diskussion

### 1.2.1 Mn(III)-Abhängigkeit des alkenspaltenden Enzyms & Reinigung des Enzyms aus dem zellfreien Extrakt

### 1.2.1.1 Mn(III)-Cofactor-Abhängigkeit der Alkenspaltungsaktivität

Im Laufe weiterer Forschungen über die intrazellulären Enzyme von *Trametes hirsuta* G FCC047 wurde überraschenderweise der Einfluss von Mn(III) auf die Alkenspaltungsaktivität entdeckt. Um zu überprüfen, ob sich der Zusatz von Mn(III) zu einer Pilzkultur mit schwacher Alkenspaltungsaktivität auf die Alkenspaltungsaktivität auswirkt, wurde eine Lösung eines Mn(III)-Salzes zum Biotransformationsreaktionsgemisch zugesetzt. Die Reaktion wurde als Dreifachansatz unter Verwendung von t-Anethol als Standardsubstrat durchgeführt. Die Probenzusammensetzung und die resultierenden Umsätze (%) sind in Tabelle 1 zusammengefasst.

**Tabelle 1: Auswirkung von Mn³⁺ auf t-Anethol mit und ohne zfE (zellfreier Pilzextrakt) als Dreifachansatz: Überführung von t-Anethol in p-Anisaldehyd nach 10 Stunden unter periodischem Zusatz von 10 µl Mn³⁺-Acetat-Lösung (1 mM)**

| **Probenzusammensetzung** | **Umsatz (%)** | | |
|---|---|---|---|
| | **1** | **2** | **3** |
| 800 µl Bis-tris-Puffer (pH 6) + 5 µl *t*-Anethol + Mn(III) | 1 | 1 | 1 |
| 800 µl zfE 1+ 5 µl *t*-Anethol + Mn(III) | 9 | 15 | 11 |
| 800 µl zfE 1+ 5 µl *t*-Anethol | 7 | 7 | 8 |
| 800 µl Bis-tris-Puffer (pH 6) +5 µl *t*-Anethol (negative Kontrolle) | 0,7 | 0,6 | 0,6 |

Aus der Studie konnte geschlossen werden, dass der Zusatz von Mn(III) zum zellfreien Extrakt zweifellos Einfluss auf die Alkenspaltungsaktivität hat, da der Umsatz (%) von *t*-Anethol zu *p*-Anisaldehyd deutlich anstieg.

### 1.2.1.2 Reinigung des Enzyms aus dem zellfreien Extrakt von Trametes hirsuta

### 1.2.1.2.1 Informationen von früher durchgeführten Reinigungen

Die Reinigung des Enzyms wurde bereits zuvor mehrere Male versucht. Obwohl es nicht gereinigt werden konnte, weil nach zwei Reinigungsschritten die Aktivität immer verloren gegangen war, konnten doch einige wertvolle Informationen gewonnen werden. Als erster Reinigungsschritt wurde immer hydrophobe Wechselwirkungschromatographie eingesetzt (Phenylsepharose CL-4B). Nach diesem Schritt war das Enzym immer noch aktiv. Es handelt sich hierbei um einen hervorragend geeigneten Vorgang, da mit den Fraktionen, in denen die Enzymaktivität vorhanden war, durchführbar und außerdem wiederholbar war. Früher wurde anschließend Größenausschlusschromatographie als zweiter Schritt eingesetzt. Nach diesem Schritt war kaum mehr Aktivität vorhanden. Ausgehend von den wenigen Fraktionen, die geringe Aktivität aufwiesen, wurde jedoch eine Enzymgröße zwischen 140-160 kDa vorhergesagt. Die positiven Fraktionen aus dem ersten Schritt (HIC) wurden außerdem ICP-MS-Unter-suchungen unterzogen, um die vorhandenen Metalle zu detektieren. Nur drei Metalle wurden gefunden: Mn, Cu und Mo. Es wurde vermutet, dass ein oder mehrere Schlüsselmetalle während des Reinigungsvorgangs verloren gehen und der Aktivitätsverlust darauf zurückzuführen ist. Eine genauere Untersuchung des Mechanismus zeigte, dass an der Enzymaktivität ein Metall beteiligt ist, das zu einem Elektronentransfer in der Lage ist. Außerdem wurde getestet, ob die Aktivität des Enzyms durch den Zusatz verschiedener Metalle (Cu¹⁺, Cu²⁺, Mn²⁺, Mo³⁺ und Mo⁵⁺) wiederhergestellt werden konnte. Alle aus der Literatur bekannten alkenspaltenden Enzyme weisen entweder Eisen oder Kupfer auf. Keines der getesteten Metalle in unterschiedlichen Oxidationszuständen konnte die verlorene Aktivität wiederherstellen. Dies war ein weiterer Hinweis darauf, dass das zuvor noch nicht getestete Mn(III) der an der Alkenspaltungsaktivität beteiligte Cofaktor sein könnte.

### 1.2.1.2.2 Herstellung eines zellfreien Extrakts und Säule

Ein zellfreier Extrakt wurde unter Verwendung einer gefriergetrockneten *T.-hirsuta-*Kultur hergestellt. Eine geeignete Konzentration der Kultur (44 mg gefriergetrocknete Kultur/ml Puffer) wurde einem Zellaufschluss mittels Ultraschallbehandlung unterzogen. Die Zelltrümmer wurden aus dem Rohextrakt entfernt, und der Überstand wurde gereinigt. Die im ersten Schritt eingesetzte Säule wurde selbst gepackt (Phenylsepharose CL-4B). Für die restlichen Schritte wurden im Handel erhältliche vorgepackte Säulen verwendet.

### 1.2.1.2.3 Hydrophobe Wechselwirkungschromatographie (Schritt 1)

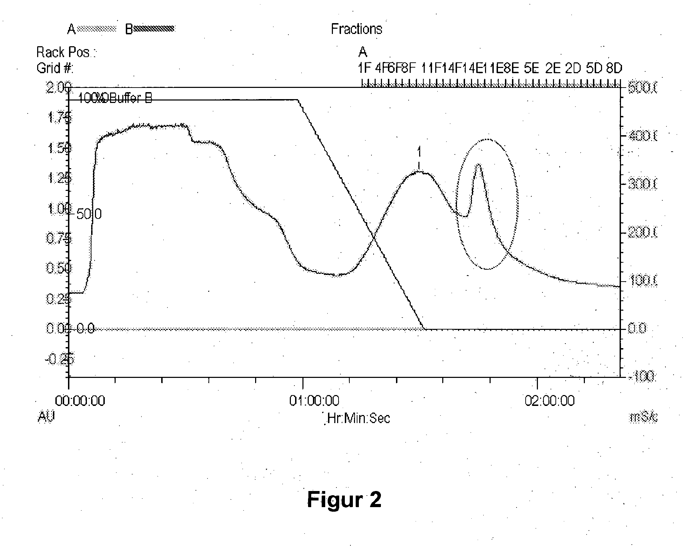

Die selbst gepackte Phenylsepharosesäule wurde eingesetzt. Dieser Schritt war wiederholbar, und die Enzymaktivität wurde immer in jenen Faktionen detektiert, die dem zweiten Peak entsprachen, der nach Ende des Salzgradienten (Ammoniumsulfat) in Wasser auftrat. Das erhaltene Chromatogramm ist in Fig. 2 zu sehen.

Der eingekreiste Bereich in Fig. 2 ist jener, in dem immer Enzymaktivität zu finden ist (zweiter Peak nach Ende des Salzgradienten). Die diesem Bereich entsprechenden Fraktionen wurden für den nächsten Reinigungsschritt gepoolt.

### 1.2.1.2.4 Anionenaustauschchromatographie (Schritt 2)

In diesem Schritt wurde die im Handel erhältliche Anionenaustauschsäule HiTrap^{®} FF Q (1 ml) verwendet. Die gepoolten positiven Fraktionen aus dem vorherigen Schritt (45 ml) wurden zur weiteren Reinigung auf die Säule geladen. Positive Fraktionen wurden durch Zusatz von kleinsten Mengen (0,4 mM) Mn(III)-Acetat getestet, was die Alkenspaltung alleine nicht katalysierte. Durch diese Salzzugabe wurde erstmals enzymatische Aktivität nach dem zweiten Reinigungsschritt beobachtet.

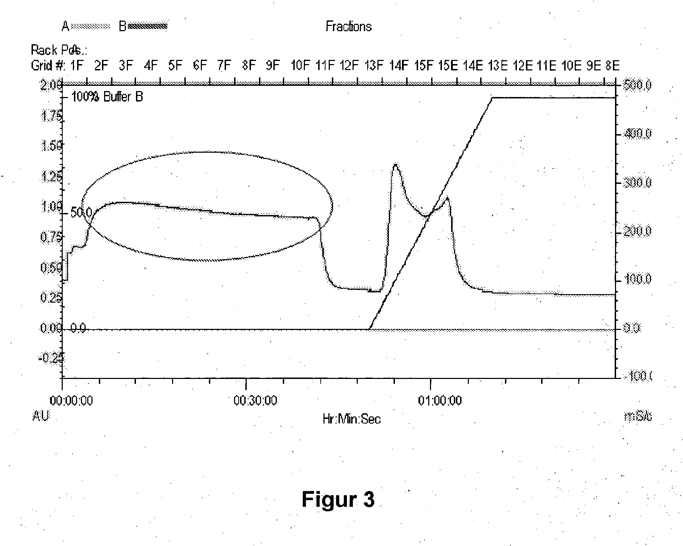

Das Enzym wurde aus den Durchflussfraktionen eluiert, was bedeutet, dass das Enzym nicht an die Säule band. Die anderen Proteine, die an die Säule banden, wurden mit einem ansteigenden linearen Salzkonzentrationsgradienten (NaCl) eluiert. Proben der Fraktionen wurden für eine Bewertung der Proteinkonzentration und für SDS-PAGE gezogen. Die positiven Fraktionen (Durchfluss) wurden dann für die weitere Reinigung gepoolt. Das Chromatogramm ist in Fig. 3 zu sehen. Die mittlere Proteinkonzentration der Durchflussfraktionen (2 bis 11) betrug 428 µg Protein/ml. Der eingekreiste Bereich ist jener, in dem die Enzymaktivität auftrat (Durchflussfraktiönen).

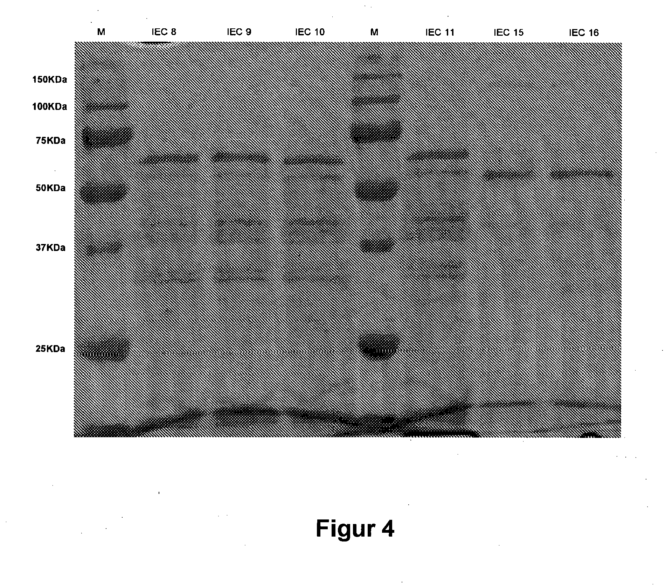

In Fig. 4 ist das Elektropherogramm einer SDS-PAGE der Anionenaustauschchromatographie-Proben wiedergegeben. Die Fraktionen 8, 9, 10 und 11 waren die aktiven Fraktionen (Durchfluss). Die Fraktionen 15 und 16 waren negative Fraktionen.

### 1.2.1.2.5 Hydrophobe Wechselwirkungschromatographie (Schritt 3)

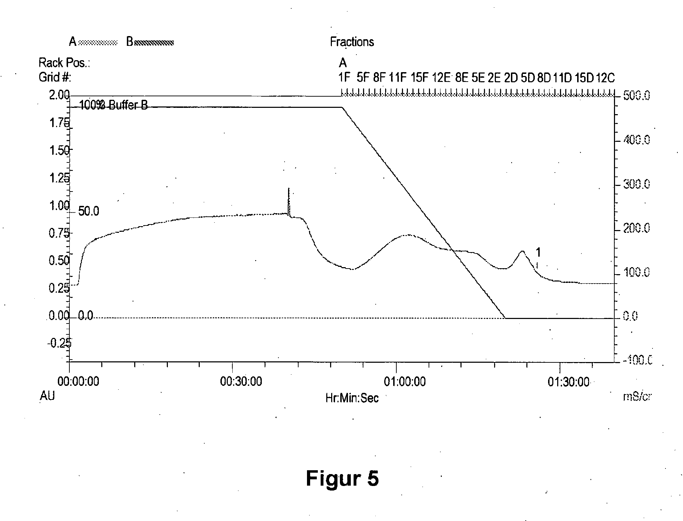

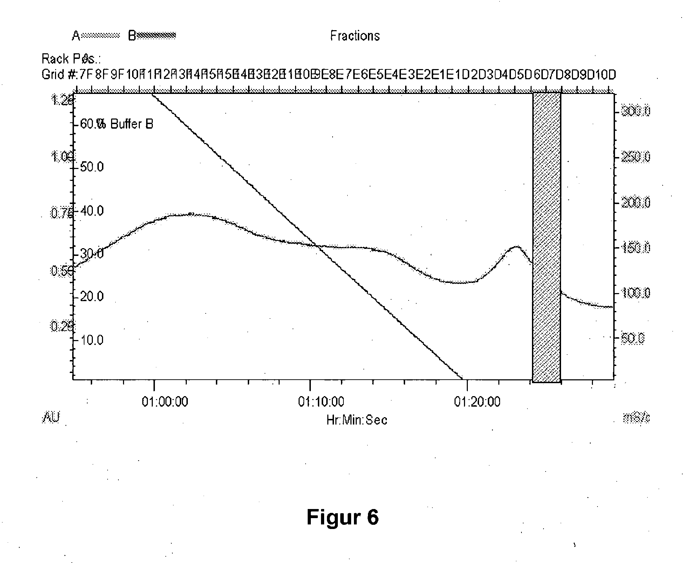

Für den letzten Reinigungsschritt wurde die im Handel erhältliche hydrophobe Wech-selwirkungschromatographie-Säule Hitrap^{®} Phenyl HP (1 ml) verwendet. Schritt 1 und 2 wurden zweimal durchgeführt, um eine ausreichende Probenmenge für Schritt 3 (den letzten Schritt) zu erhalten. Die vereinigte Probe aus der Anionenaustauschchromatographie (zweimal durchgeführt, ergab ein Volumen von etwa 80 ml mit etwa 428 µg Protein/ml) wurde auf die HIC-Säule geladen. Als der Versuch zum ersten Mal durchgeführt wurde, wurden alle Fraktionen auf Aktivität getestet. Damit war klar, wo das Enzym von Interesse eluierte. Der Bereich war ähnlich wie jener, in dem es bei der hydrophoben Wechselwirkungschromatographie in Schritt 1 eluierte (nach Ende des Ammoniumsulfatgradienten, in Wasser). Als der gesamte Versuch ein zweites Mal wiederholt wurde, wurde ein Teil der Fraktionen (700 µl) nach dem Ammoniumsulfat-Konzentrationsgradienten gewonnen und auf Aktivität getestet, um die aktiven Fraktionen zu identifizieren. Die restliche Menge der Fraktionen wurde für eine Beurteilung der Proteinkonzentration und für SDS-PAGE gelagert. Fraktion 36 wies die stärkste Aktivität auf (16 % Umsatz von *t*-Anethol zu p-Anisaldehyd unter den Reaktionsbedingungen). Sie wies eine Proteinkonzentration von etwa 150 µg Protein/ml auf. Das erhaltene Chromatogramm ist in Fig. 5 zu sehen. Die Enzymaktivität wurde in zwei Fraktionen nach Ende des Ammoniumsulfat-Konzentrationsgradienten gefunden. Fig. 6 ist eine vergrößerte Ansicht des Chromatogramms auf Fig. 5. Der schraffierte Bereich zeigt die Fraktionen an, in denen Enzymaktivität gefunden wurde (Fraktionen 36 und 37).

### 1.2.1.3 SDS-PAGE der aktiven Fraktion und Aminosäuresequenzanalyse

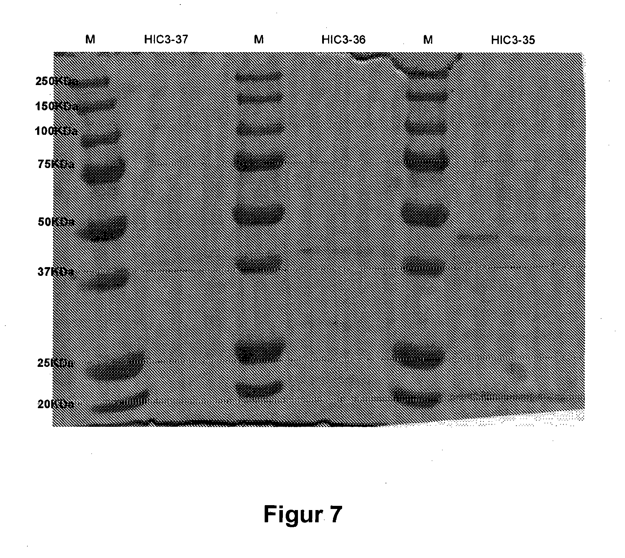

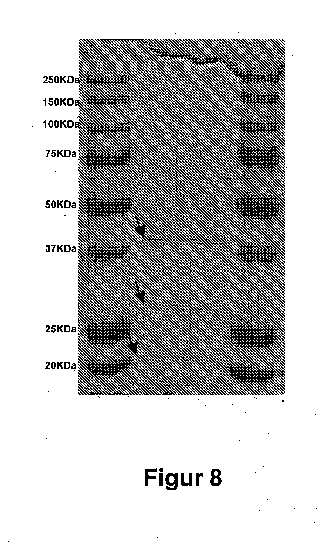

SDS-PAGE der aktiven Fraktion ergab drei Hauptbanden. Eine im Bereich von 37 kDa, eine im Bereich von 25 kDa und die letzte im Bereich von 20 kDa. Fig. 7 ist das Elektropherogramm der Fraktionen 35, 36 und 37 der Reinigung durch hydrophobe Wechselwirkungschromatographie als Reinigungsschritt 3. Fraktion 36 war die aktivste Fraktion. Da die Proteinkonzentration der Fraktionen niedrig war, wurden zwei Vertiefungen im Gel verbunden, um eine größere Vertiefung zu erhalten, und 80 µl Probe (Fraktion: Ladepuffer = 50:50) wurden in jede Vertiefung gefüllt. Der Vergleich zwischen der positiven Fraktion (HIC3-36) und der negativen Fraktion (HIC3-35) in Fig. 7 lässt darauf schließen, dass die Bande im Bereich von 37 kDa eine einzigartige Bande der positiven Fraktion ist. In Fig. 8 ist ein Elektropherogramm der aktivsten Fraktion 36 zu sehen. Die Bande nahe bei 37 kDa wurde aus dem Gel ausgeschnitten und für eine MALDI-MS-MS-Analyse und De-Novo-Sequenzierung an die PROTAGEN AG in Dortmund, Deutschland geschickt.

### 1.2.2 Aminosäuresequenzierung der erhaltenen SDS-Gelbande

Der folgende Abschnitt ist zum Teil eine Kopie der Daten und des Berichts der PROTAGEN AG.

### 1.2.2.1 Protein-Identifikation mittels MALDI-MS/MS und De-Novo-Sequenzierung

### 1.2.2.1.1 In-Gel-Verdau

Der ausgewählte Protein-Spot wurde aus dem Gel ausgeschnitten. Das Gelstück wurde abwechselnd dreimal mit 15 µl 10 mM NH₄HCO₃ und 5 mM NH₄HCO₃/50 % Acetonitril gewaschen. Nachdem das Gelstück getrocknet worden war, wurde eine Trypsin-Lösung (33 ng/µl in 10 mM NH₄HCO₃, pH 7,8) zugesetzt, um das Protein mehrere Stunden lang bei 37 °C zu verdauen.

### 1.2.2.1.2 MALDI-Probenvorbereitung

Die Peptide wurden mit 0,1 % Trifluoressigsäure (TFA) aus dem Gelstück extrahiert und unter Verwendung von C18-Material (ZipTip™, Millipore, Bedford, MA, USA) gereinigt, bevor sie als Spots auf das MALDI-Target aufgetragen wurden.

### 1.2.2.1.3 Erhalt des MALDI-Spektrums

Die Proteinidentifizierung erfolgte unter Verwendung eines Massenspektrometers Ultraflex III TOF/TOF (Bruker Daltonics). Um Peptidmassen-Fingerprintspektren (PMF, MS) zu erhalten, wurden 200 Single-Shot-Spektren gemittelt, und Peak-Picking erfolgte mithilfe des SNAP-Algorithmus. Die resultierende Massenliste wurde zur Proteinidentifizierung an die Datenbank ProteinScape™ gesandt. Sofern möglich, wurden Peptidfragmentspektren (PFF, MS/MS) erhalten. Die Peaks zur Fragmentierung wurden unter Verwendung der ProteinScape-Datenbank basierend auf den Ergebnissen der Proteinidentifierung mittels PMF ausgewählt.

### 1.2.2.1.4 Proteinidentifizierung unter Verwendung einer öffentlichen Datenbank

Die Proteinidentifizierung erfolgte, indem die Massenspektren mithilfe verschiedener externer Suchalgorithmen ((ProFound™, Mascot™, Sequest™) in der NCBI-Proteindatenbank (http://www.ncbi.nlm.nih.gov/) gesucht wurden. Für PMF-Spektren wurde die Massentoleranz auf 50 ppm eingestellt. Die Proteinidentifizierung basierte auf dem Metascore1, der aus den einzelnen Suchergebnissen der ProteinScape™-Datenbank berechnet wurde, und erforderlichenfalls auf manueller Überprüfung der Daten. PFF-Spektren wurden entweder verwendet, um das bereits durch PMF identifizierte Protein zu bestätigen oder um Proteine zu identifizieren, die der PMF-Identifizierung entgangen waren.

### 1.2.2.1.5 Automatisierte De-Novo-Sequenzierung

Die MS/MS-Datensätze wurden unter Verwendung des Softwarepakets PEAKS 4.5 einer automatischen De-Novo-Sequenzierung unterzogen. Für jedes MS/MS-Spektrum werden die Sequenzen mit den höchsten Scores angeführt.

### 1.2.2.1.6 Datenbanksuche

Das folgende Protein wurde mittels einer Datenbanksuche identifiziert: gi|170091822|ref|XP_001877133.1| Asparaginsäure-Peptidase A1 [Laccaria bicolor S238NH82]. Das Peptid EPGLAFAFGK konnte mittels einer Datenbanksuche identifiziert und diesem Protein zugeordnet werden.

### 1.2.2.1.7 De-Novo-Sequenzierung

### Probe PG379-U11-2010-001

Die folgenden Peptidsequenzen wurden mittels De-Novo-Sequenzierung erhalten. Die fett dargestellten Aminosäuren wurden mit sehr hohem Konfidenzwert identifiziert (PEAKS-Score dieses Sequenzteils >90 %).

| Ausgangsmasse | Peptid |
|---|---|
| 1036,5482 | E P G L A F A F G K* |
| 1166,6215 | L V D S P V F S F R |
| 1301,6235 | K Y Y T V Y D H G R |
| 2041,0100 | N Q D F A E A T K E P G L A F A F G K |
| 1173,5321 | Y Y T V Y D H G R |
| 1366,6850 | A Y W E V E L E S I K |
| 2375,0981 | L G S S E E D G G E A L F G G V D E TA Y S G K |
| 1023,4805 | N Q D F A E A T K |
| 1494,7802 | K A Y W E V E L E S I K |

| | |
|---|---|
| * ebenfalls mittels Datenbanksuche gefunden | |

Diese Peptide wurden durch De-Novo-Sequenzierung identifiziert und konnten, aufgrund der hohen Sequenzhomologie zwischen dem durch De-Novo-Sequenzierung identifizierten Peptid und einer Sequenzregion von gi|17009182, gi|17009182 zugeordnet werden. Somit wird davon ausgegangen, dass alle Peptide Teil desselben Proteins sind.

### 1.2.3 Identifizierung der Gensequenz des alkenspaltenden Enzyms

### 1.2.3.1 PCR unter Verwendung von konstruierten degenerierten Primern und

### cDNA von Trametes hirsuta als Matrize

### 1.2.3.1.1 Anfängliche PCR-Reaktionsoptimierung

Eine PCR wurde unter Verwendung verschiedener Kombinationen von konstruierten degenerierten Vorwärts- und Rückwärtsprimern durchgeführt. Ausgehend von der Kartierung der erhaltenen Sequenzen mit der Aminosäuresequenz der Asparaginsäure-Peptidase A1 von *Laccaria bicolor,* wurden drei Vorwärts- und zwei Rückwärtsprimer konstruiert. Unter Verwendung derselben wurden sechs unterschiedliche Kombinationen von Vorwärts- und Rückwärtsprimern hergestellt. Um zu überprüfen, welche Temperatur für die PCR geeignet ist, wurden drei Temperaturen unterhalb des Schmelzpunkts der Primer getestet. Die unterschiedlichen Primerkombinationen sind in Tabelle 2 angeführt. Die eingesetzten PCR-Bedingungen sind in Tabelle 3 zusammengefasst. Das Programm der PCR-Reaktionen ist in Tabelle 4 angeführt.

### Legende:

| | |
|---|---|
| Vorwärts-Primer 1: AAY CAR GAY TTY GCN GAR GC | - **NQDFAEA** |
| Vorwärts-Primer 2: GAR GAR GAY GGN GGN GAR GCN | - **EEDGGEA** |
| Vorwärts-Primer 3: AAR GCN TAY TGG GAR GTN GA | - **KAYWEVE** |
| Rückwärts-Primer 1: TC NAC YTC CCA RTA NGC YTT | **- KAYWEVE** |
| Rückwärts-Primer 2: C RTG RTC RTA NAC NGT RTA RTA YT | - **KYYTVYDHG** |

**Tabelle 2: Probennummerierung der unterschiedlichen Kombinationen degenerierter Vorwärts- und Rückwärts-Primer**

| **Probe Nr.** | **Primerkombination** |
|---|---|
| 1 | Vorwärts-Primer 1 und Rückwärts-Primer 1 |
| 2 | Vorwärts-Primer 1 und Rückwärts-Primer 2 |
| 3 | Vorwärts-Primer 2 und Rückwärts-Primer 1 |
| 4 | Vorwärts-Primer 2 und Rückwärts-Primer 2 |
| 5 | Vorwärts-Primer 3 und Rückwärts-Primer 1 |
| 6 | Vorwärts-Primer 3 und Rückwärts-Primer 2 |

**Tabelle 3: Reaktionskomponenten für PCR unter Verwendung der unterschiedlichen Primerkombinationen**

| **Inhalt** | **Primerkombinationen (1-6)** | | |
|---|---|---|---|
| | **a (47,1 °C) (µl)** | **b (50,2 °C) (µl)** | **c (53 °C) (µl)** |
| Puffer (5X HF) | 10 | 10 | 10 |
| dNTPs (10mM) | 1 | 1 | 1 |
| Vorwärts-Primer (1:10 verdünnt) | 1 | 1 | 1 |
| Rückwärts-Primer (1:10 verdünnt) | 1 | 1 | 1 |
| cDNA (180,5 ng/µl) | 1 | 1 | 1 |
| steriles Wasser | 35,5 | 35,5 | 35,5 |
| DNA-Polymerase (Phusion) | 0,5 | 0,5 | 0,5 |

**Tabelle 4: PCR-Programm zur Untersuchung unterschiedlicher Temperaturen mit degenerierten Primern**

| **Schritt** | **Temperatur (°C)** | **Dauer** | **Zyklenanzahl** |
|---|---|---|---|
| Anfangsdenaturierung | 98 | 2 min | 1 |
| Denaturierung | 98 | 30 s | |
| Annealing | T = 50 | 30 s | 40 |
| | Gradient = 3 °C | | |
| | Rate = 3 °C/s | | |
| Extension | 72 | 45 s | |
| Endextension | 72 | 7 min | 1 |

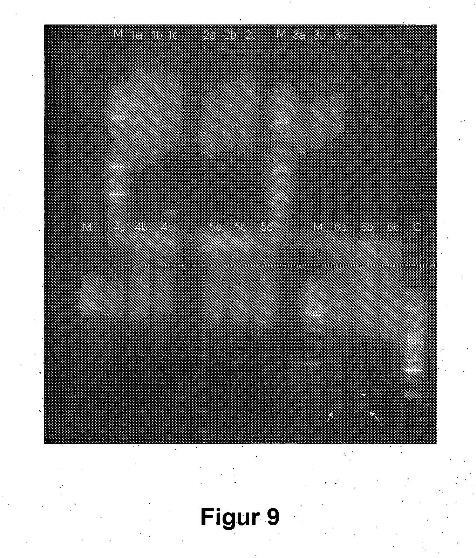

Nach Durchführung der PCR wurden die Proben (jeweils 50 µl) präparativ auf Agarosegel (1 % Agarose) analysiert. Drei Banden waren erkennbar (1c, 6a und 6b). Alle Produkte waren etwa 200 bp groß. Ein Bild des Agarosegels ist in Fig. 9 zu sehen. Ein PCR-Produkt wurde für die Kombinationen 1 c, 6a und 6b erhalten, wie dies in Fig. 9 mit Pfeilen markiert ist.

### 1.2.3.1.2 Extraktion und Amplifikation von PCR-Produkten

Die Extraktion der DNA aus den Banden, die aus dem Gel ausgeschnitten worden waren, erfolgte mithilfe des Gelextraktionssets QIAquick^{®} (50) von QIAGEN. Die DNA-Konzentration der drei Proben (Volumen: 50 µl) wurde gemessen. Der Elutionspuffer aus dem Set wurde als Blindprobe verwendet:
1c - 8,9 ng/µl (etwa 200 bp)
6a - 7,6 ng/µl (etwa 200 bp)
6b - 7,8 ng/µl (etwa 200 bp).

Die DNA-Fragmente wurden kloniert und dann zur Amplifikation der DNA in TOP-10-*E.-coli*-Zellen transformiert. Das Klonen der DNA-Fragmente (1c, 6a und 6b) erfolgte unter Verwendung des PCR-Kloniersets CloneJET™. Die Menge an DNA-Probe, die von jeder Probe entnommen wurde, basierte auf der Größe des Fragments und der Konzentration der gereinigten DNA-Probe. Das Verfahren folgte dem Handbuch des Sets.

Übernacht-Kulturen (5 ml LB + 100 µg/l Amp in jedem 50-ml-Röhrchen) der Transformanten wurden gestartet (von jeder Transformantenplatte wurden 3 Kolonien genommen). Insgesamt wurden 9 Röhrchen bei 37 °C unter Rütteln bei 120 U/min über Nacht inkubiert. Ein Backup jeder der Kolonien wurde auch auf Agarplatten durchgeführt (LB + 100 µg/l Amp). Die Platten wurden am nächsten Tag entnommen und bei 4 °C gelagert.

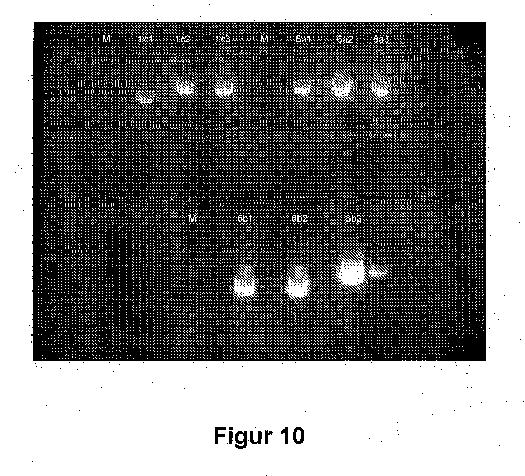

Das Spin-Minipräparationsset QIAprep^{®} (250) von QIAGEN wurde zur Durchführung einer Minipräparation verwendet. Der Vorgang erfolgte gemäß dem Handbuch. Die aus den Minipräparaten eluierte DNA (jeweils 50 µl) wurde mit 6X Ladepuffer (8,3 µl) vermischt, und die ganze Probe wurde auf das Agarosegel (1 %) aufgegeben; siehe Fig. 10, die die amplifizierten PCR-Produkte im CloneJET™-Kloniervektor zeigt. Die Banden der Plasmide wurden aus den einzelnen Spuren ausgeschnitten und in Röhrchen gefüllt (15 ml). Die Gelstücke wurden gewogen, und die DNA wurde mithilfe des Gelextraktionssets QIAquick^{®} (50) von QIAGEN extrahiert. Die DNA-Konzentration der 9 Proben (Volumen 50 µl) wurde gemessen. Der Elutionspuffer aus dem Set wurde als Blindprobe verwendet:
1c1 - 20,7 ng/µl, 1c2 - 12,1 ng/µl, 1c3 - 22,8 ng/µl, 6a1 - 16,7 ng/µl, 6a2 - 14,3 ng/µl, 6a3 - 11,8 ng/µl, 6b1 - 25,4 ng/µl, 6b2 - 20 ng/µl, 6b3 - 61,9 ng/µl.

### 1.2.3.1.3 Analyse von Insert-Sequenzen und Design von definitiven Primern für Primer-Walking

Die Sequenzierungsergebnisse für 1 c1, 6a1 und 6b1 wurden mittels BLAST einzeln mit der Sequenz des Plasmids pJET1.2 verglichen. Die nicht identische Region wurde als Sequenz des Inserts im Plasmid identifiziert. Diese Region wurde in die Aminosäuresequenz translatiert. Mit den unterschiedlichen Leserastern wurde die Gegenwart der verwendeten Vorwärts- (fwd) und Rückwärts- (rev) Primer (fwd 1 und rev 1 im Falle von 1 c1 und fwd 3 und rev 2 im Falle von 6a1 und 6b1) überprüft. Es wurde herausgefunden, dass nur in der 6a1-Sequenz beide Primer vorhanden waren. Ein Protein-Blast mit dieser Aminosäuresequenz ergab gewisse Ähnlichkeiten mit mehreren Pilzproteinen, die zur Pepsin-Retropepsin-Überfamilie gehören. (Das zweitbeste Ergebnis war Asparaginsäure-Peptidase A1 von *Laccaria bicolor).* Die Sequenz der Probe 6a1 ist nachstehend zu sehen. Die Kriterien für den Entwurf der endgültigen Primer waren, dass beide Primer am Ende einen GC-Gehalt aufweisen und dass der Schmelzpunkt der beiden Primer ähnlich sein sollte. Der Schmelzpunkt und die Wahrscheinlichkeit von Selbstkomplementarität der Primer wurden mithilfe des Onlineprogramms Oligo-Calc überprüft. Im Falle des Rückwärts-Primers wurde das reverse Komplement des ursprünglichen DNA-Stücks hergestellt, und die Länge wurde geändert, um die Anforderungen zu erfüllen. Die Primer wurden so konstruiert, dass sie die anfängliche degenerierte Primerregion umfassten (um die Genauigkeit beim Primer-Walking genau zu überprüfen).
>6a1-Vorwärts.pJET1.2-F

Durch Translation der Insert-DNA-Sequenz erhaltene Aminosäuresequenz: >EMBOSS_001_3

Die unterstrichenen Sequenzen entsprechen jeweils einem der folgenden Primer:
degenerierter Vorwärts-Primer
degenerierter Rückwärts-Primer
Beginn der Vektorsequenz
konstruierter definitiver Vorwärts-Primer (PW-1 Fwd)
konstruierter definitiver Rückwärts-Primer (PW-1 Rev)

### 1.2.3.2 Primer-Walking 1

### 1.2.3.2.1 Design von Primern für das cDNA-Ende

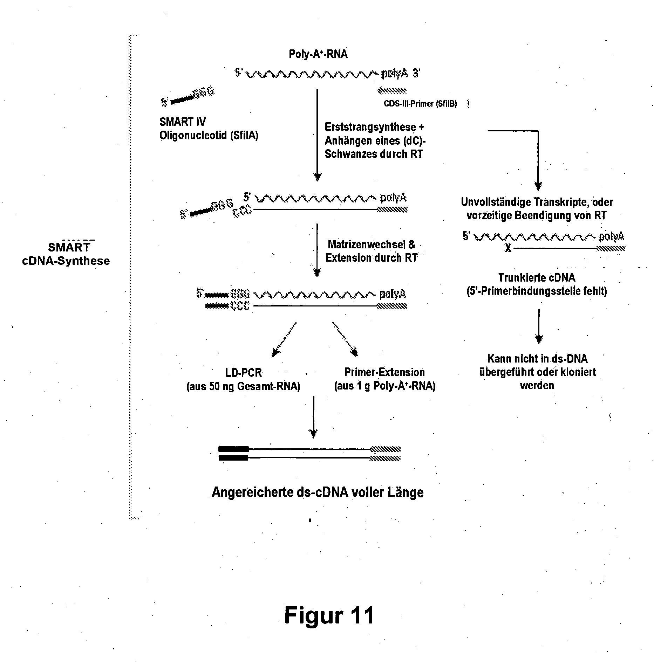

Der Versuch, eine PCR mit nur einem Primer (dem definitiven Vorwärts- bzw. dem definitiven Rückwärts-Primer) durchzuführen, ergab keinerlei PCR-Produkt. Die PCR erforderte eine Kombination aus sowohl Vorwärts- als auch Rückwärts-Primer. Um einen weiteren Satz von Vorwärts- und Rückwärts-Primern zu konstruieren, wurde auf die Konstruktion der cDNA zurückgegriffen. Das Bibliotheken-Konstruktionsset SMART^{™}DNA wurde verwendet, um die cDNA aus der mRNA von *Trametes hirsuta* zu erhalten. Es wurde der Primer CDS III für die cDNA-Erststrangsynthese verwendet, ausgehend vom Poly-A⁺-Schwanz der mRNA. Für die Zweitstrangsynthese wurde das Oligonukleotid SMART IV verwendet. Somit enthält jedes cDNA-Fragment die Sequenz des CDS-III-Primers am 3'-Ende und die Sequenz des SMART-IV-Primers am 5'-Ende (siehe Fig. 11). So wurde ein auf der Sequenz des SMART-IV-Oligo-nucleotids basierender Vorwärts-Primer und ein auf dem CDS-III-Primer basierender Rückwärtsprimer auf solche Weise konstruiert, dass beide einen ähnlichen Schmelzpunkt aufweisen wie die vorher konstruierten definitiven Vorwärts- und Rückwärts-Primer. Ein Fließbild der cDNA-Konstruktion aus dem Benutzerhandbuch des Sets ist in Fig. 11 zu sehen. Die neuen Primer wurden darauf basierend konstruiert. Die zwei konstruierten Vorwärts- und Rückwärts-Primer sind nachstehend angeführt:

| | |
|---|---|
| PW1-Fwd | - TGTGGATCATTGGTGATGTCTTCCTGC |
| PW1-Rev | - GTCTCCGAGTTTGATCGATTCCAGC |
| SMART-IV | - GTATCAACGCAGAGTGGCCATTACG |
| CDS III | - CGAGGCGGCCGACATGTTTTTTTT |

### 1.2.3.2.2 PCR-Primer-Walking 1

Unter Verwendung der unterschiedlichen Kombinationen aus Vorwärts- und Rückwärts-Primern wurde eine PCR durchgeführt. Die wichtigste Veränderung im PCR-Programm betraf die Annealing-Temperatur. Da alle Primer eine ähnliche Schmelztemperatur aufwiesen, wurden zwei unterschiedliche Temperaturen getestet. Die verschiedenen Primerkombinationen sind in Tabelle 5 angeführt. Die verschiedenen Reaktionskomponenten sind in Tabelle 6 zusammengefasst. Das PCR-Reaktionsprogramm ist in Tabelle 7 zu sehen.

**Tabelle 5: Die unterschiedlichen Primerkombinationen und Probennummerierung**

| **Probe Nr.** | **Primerkombination** |
|---|---|
| 1 | Pw1-Fwd und SMART IV fwd |
| 2 | Pw1-fwd und CDS III rev |
| 3 | Pw1-rev und SMART IV fwd |
| 4 | Pw1-rev und CDS III rev |

**Tabelle 6: Reaktionskomponenten für eine PCR unter Verwendung der unterschiedlichen Primerkombinationen für Primer-Walking 1**

| **Inhalt** | **Primerkombination (1-4)** | |
|---|---|---|
| | **a (59,3 °C) (µl)** | **b (63,4 °C) (µl)** |
| Puffer (5X HF) | 10 | 10 |
| dNTPs (10mM) | 1 | 1 |
| Vorwärts-Primer (1:10 verdünnt) | 1 | 1 |
| Rückwärts-Primer (1:10 verdünnt) | 1 | 1 |
| cDNA (180,5 ng/µl) | 1 | 1 |
| steriles Wasser | 35,5 | 35,5 |
| DNA-Polymerase (Phusion) | 0,5 | 0,5 |

**Tabelle 7: PCR-Programm zur Untersuchung unterschiedlicher Temperaturen für Primer-Walking 1**

| **Schritt** | **Temperatur (°C)** | **Dauer** | **Zyklenanzahl** |
|---|---|---|---|
| Anfangsdenaturierung | 98 | 2 min | 1 |
| Denaturierung | 98 | 30 s | 40 |
| Annealing | T=60 Gradient = 4 °C Rate = 3 °C/s | 45 s | |
| Extension | 72 | 45 s | |
| Endextension | 72 | 7 min | 1 |

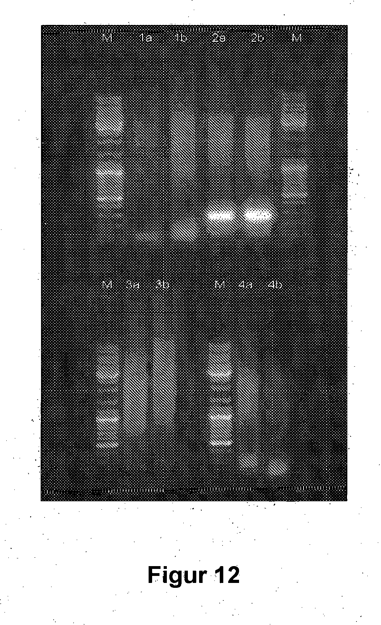

Nach Durchführung der PCR wurden die Proben (jeweils 50 µl) präparativ auf Agarosegel (1 % Agarose) analysiert. Bei Kombination Nr. 2 waren bei beiden getesteten Temperaturen klare Banden erkennbar (etwa 300 bp). Für Kombination Nr. 3 waren bei beiden Temperaturen schwache Banden erkennbar (etwa 300 bp). Kombination 2 und 3 waren die empfindlichen Kombinationen, da nur ein Vorwärts- und ein Rückwärts-Primer vorhanden waren. Einige andere Produkte wurden ebenfalls erhalten, aber ihre Größe war gering, weshalb sie nicht ausgeschnitten wurden. Das Agarosegel ist in Fig. 12 zu sehen. Bei Primer-Walking 1 wurden somit PCR-Produkte für die Kombinationen 2a, 2b, 3a und 3b erhalten. Die Extraktion und Amplifikation der PCR-Produkte 2a, 2b, 3a und 3b erfolgte auf ähnliche Weise wie in Abschnitt 1.2.3.2.3 beschrieben.

### 1.2.3.2.3 Sequenzanalyse für Primer-Walking 1

Von allen sequenzierten Proben wurden für eine der 2a-Insert-Proben und zwei der 3b-Insert-Proben klare Ergebnisse erhalten. Da die Sequenzierung sowohl in Vorwärts- als auch Rückwärtsrichtung erfolgen kann, wurden die Sequenzen auf die tatsächliche Primersequenz sowie auf die reverse komplementäre Sequenz der Primer überprüft:

| | |
|---|---|
| PW1-Fwd | - TGTGGATCATTGGTGATGTCTTCCTGC |
| CDS III | - CGAGGCGGCCGACATGTTTTTTTT |
| PW1-Rev | - GTCTCCGAGTTTGATCGATTCCAGC |
| SMART-IV | - GTATCAACGCAGAGTGGCCATTACG |
| PW1-Fwd rev | - GCAGGAAGACATCACCAATGATCCACA |
| CDS III rev | - AAAAAAAACATGTCGGCCGCCTCG |
| PW1-Rev rev | - GCTGGAATCGATCÄAACTCGGAGAC |
| SMART-IV rev | - CGTAATGGCCACTCTGCGTTGATAC |

>PW-1-2a3.pJET1.2-F Translation des Inserts 2a3:
>EMBOSS_001_3
>PW-1-3b2.pJET1.2-F
Translation des Inserts 3b1

Die Analyse der Sequenz zeigte, dass das Insert 2a sowohl die PW-I-Vorwärts-Primersequenz als auch die ursprüngliche degenerierte Rückwärts-Primersequenz aufwies. Somit ist sie Teil des Proteins von Interesse. Die Translation der Sequenz ergab eine Sequenz mit einem Stoppcodon dazwischen. Folglich war das Ende des Proteins von Interesse erreicht. Im Falle der Sequenz des Inserts 3b war der PW-I-Rückwärts-Primer vorhanden, aber er umfasste nicht die anfängliche degenerierte Vorwärts-Primersequenz. Somit war klar, dass der konstruierte Rückwärts-Primer PW-1- rev nicht spezifisch genug war und an einen anderen Teil der cDNA gebunden hatte. Daher wurden drei andere Rückwärts-Primer konstruiert, die für Primer-Walk-ing 2 verwendet wurden. Insgesamt konnten 84 Aminosäurereste am Ende von Primer-Walking 1 abgeleitet werden, und das Ende des Proteins war erreicht worden. Die Sequenz des bis zu diesem Schritt erhaltenen Peptids ist nachstehend angeführt:

### 1.2.3.3 Primer-Walking 2

### 1.2.3.3.1 Design von alternativen Rückwärts-Primern

Da der Rückwärts-Primer (PW-1 rev) aus Primer-Walking 1 nicht spezifisch genug für die PCR war, mussten alternative Rückwärts-Primer konstruiert werden. Drei alternative Rückwärts-Primer wurden konstruiert. Weiters wurde (basierend auf der BLAST-Suche) bemerkt, dass die Rückwärts-Primer am Ende der gesamten Proteinsequenz lagen. Folglich wurde der degenerierte Primer (Fwd-1) aus dem ersten Schritt (von dem bei der BLAST-Suche erwartet wurde, dass er inmitten der Sequenz liegt) als Vorwärts-Primer verwendet.
>6a1-Vorwärts.pJET1.2-F (Ergebnis von 2.1) Die reversen Komplemente der oben markierten Sequenzen
1) PW-2 rev Pri-1: AGCTCAAGCTCGTCGTCTCCG
2) PW-2 rev Pri-2: AGTGTCGATGGCAGCGCCG
3) PW-2 rev Pri-3: GATTTGCACATTGAGCATCTCCGCC

### 1.2.3.3.2 PCR-Primer-Walking 2

Es wurde eine PCR unter Verwendung der Kombinationen der drei neuen Rückwärts-Primer, die mit dem degenerierten Vonwärts-Primer (Fwd-1) konstruiert worden waren, versucht. Versuchsweise wurde auch einer der Rückwärts-Primer mit dem an das Ende der cDNA bindenden Primer (SMART IV fwd) überprüft, um zu sehen, ob klare Banden erhalten werden können (aufgrund des großen Abstands zum Beginn des Proteins). Zur neuerlichen Überprüfung wurde die Kombination aus degeneriertem Vorwärts-Primer (Fwd-1) und degeneriertem Rückwärts-Primer (Rev-2) aus Schritt 1 ebenfalls wiederholt. Die unterschiedlichen Primerkombinationen, die verwendet wurden, sind in Tabelle 8 angeführt. Die PCR-Komponenten sind in Tabelle 9 zusammengefasst, und das verwendete PCR-Programm ist in Tabelle 10 zu sehen.

**Tabelle 8: Nummerierung der unterschiedlichen Primerkombinationen, die für PW-2 getestet wurden.**

| **Probe Nr.** | **Primerkombination** |
|---|---|
| 1 | degenerierter Fwd-1 und degenerierter Rev-1 |
| 2 | SMART IV Fwd und PW-2-Rev-Pri-2 |
| 3 | degenerierter Fwd-1 und PW-2-Rev-Pri-1 |
| 4 | degenerierter Fwd-1 und PW-2-Rev-Pri-2 |
| 5 | degenerierter Fwd-1 und PW-2-Rev-Pri-3 |

**Tabelle 9: Reaktionskomponenten für die PCR unter Verwendung der unterschiedlichen Primerkombinationen für Primer-Walking 2**

| **Inhalt** | **Primerkombination (1-5)** | | |
|---|---|---|---|
| | **A (53,2 °C) (µl)** | **b (56,5 °C) (µl)** | **c (59,4 °C) (µl)** |
| Puffer (5X HF) | 10 | 10 | 10 |
| dNTPs (10mM) | 1 | 1 | 1 |
| Vorwärts-Primer (1:10 verdünnt) | 1 | 1 | 1 |
| Rückwärts-Primer (1:10 verdünnt) | 1 | 1 | 1 |
| cDNA (180,5 ng/µl) | 1 | 1 | 1 |
| steriles Wasser | 35,5 | 35,5 | 35,5 |
| DNA-Polymerase (Phusion) | 0,5 | 0,5 | 0,5 |

**Tabelle 10: PCR-Programm zur Untersuchung verschiedener Temperaturen für Primer-Walking 2**

| **Schritt** | **Temperatur (°C)** | **Dauer** | **Zyklenanzahl** |
|---|---|---|---|
| Anfangsdenaturierung | 98 | 2 min | 1 |
| Denaturierung | 98 | 30 s | |
| Annealing | T = 55 | 45 s | 40 |
| | Gradient = 4 °C | | |
| | Rate = 3 °C/s | | |
| Extension | 72 | 1 min | |
| Endextension | 72 | 7 min | 1 |

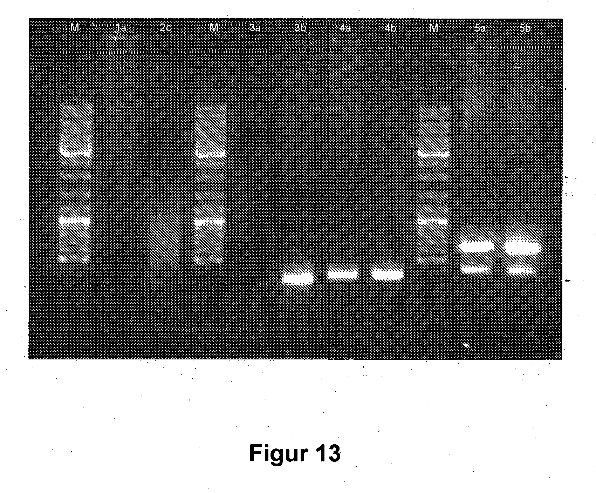

Nach der Durchführung der PCR wurden die Proben (jeweils 50 µl) präparativ auf Agarosegel (1 % Agarose) analysiert, wie in Fig. 13 dargestellt. Klare Banden (PCR-Produkte) waren bei etwa 400 bp für die Proben 3b, 4a, 4b, 5a und 5b erkennbar. Außerdem waren für die Kombinationen 5a und 5b auch ausgeprägte Banden bei etwa 600 bp erkennbar. In Kombination Nr. 2 waren jedoch keine ausgeprägten Banden zu erkennen. Es ist möglich, dass die Entfernung zwischen dem 5'-Ende der cDNA und dem Rückwärts-Primer zu groß war. Es ist auch möglich, dass die Dauer des DNA-Strangextensionsschritts für die große Entfernung für eine vollständige Extension zu kurz war.

Die Extraktion und Amplifikation der PCR-Produkte 3b, 4a, 4b, 5a-kleine Bande, 5b-kleine Bande, 5a-große Bande und 5b-große Bande erfolgten ähnlich wie in Abschnitt 1.2.3.1.2 beschrieben. Unglücklicherweise waren die Transformationsplatten von 3b, 4a und 5a-große Bande kontaminiert. Folglich wurden nur die Proben 4b, 5a-kleine Bande, 5b-kleine Bande und 5b-große Bande sequenziert.

### 1.2.3.3.3 Sequenzanalyse für Primer-Walking 2

Die erhaltenen DNA-Sequenzen wurden analysiert, um die Primer und somit die Sequenz der Inserts zu identifizieren. Alle Inserts führten zur selben Sequenz. Die Identität der erhaltenen Sequenzen wurden verglichen. Es zeigte sich, dass die Sequenzen vollständig identisch waren.

### Legende:

**PW-2-Rückwärts-Primer**
   1) PW-2 rev Pri-1: AGCTCAAGCTCGTCGTCTCCG
   2) PW-2 rev Pri-2: AGTGTCGATGGCAGCGCCG
   3) PW-2 rev Pri-3: GATTTGCACATTGAGCATCTCCGCC
**Reverses Komplement der Rückwärts-Primer:**
   2) PW-2 rev Pri-2: CGGCGCTGCCATCGACACT
   3) PW-2 rev Pri-3: GGCGGAGATGCTCAATGTGCAAATC
**degenerierter Fwd-1-Primer**
   AAYCARGAYTTYGCNGARGC

>4b1.pJET1.2-F
>Translation des Inserts 4b1
>5a1 kleineBande.pJET1.2-F
> Translation des Inserts 5a1-kleine Bande
>5b2großeBande.pJET1.2-F
> Translation des Inserts 5b2-große Bande

### Proteinsequenz nach Primer-Walking-Schritt 1:

>EMBOSS_001_3

### Proteinsequenz aus Primer-Walking-Schritt 2:

Primer-Walking 2 unter Verwendung der neu konstruierten Rückwärts-Primer und der degenerierten Vorwärts-Primer war erfolgreich. Die Sequenz konnte leicht verifiziert werden, da sie mehrere zuvor konstruierte Primersequenzen enthielt. 89 weitere Aminosäurereste wurden bei Primer-Walking 2 bestimmt. Nach Primer-Walking 2 waren 229 Aminosäurereste des Proteins bestimmt, und die halbe Strecke des Proteins war erreicht (basierend auf einer BLAST-Ähnlichkeitssuche). Die Proteinsequenz nach Primer-Walking 2 ist nachstehend angeführt:

### 1.2.3.4 Primer-Walking 3

### 1.2.3.4.1 Design von Primern für Primer-Walking 3

Die ersten beiden Primer-Walking-Schritte waren erfolgreich. Im nächsten Schritt wurde die Situation etwas schwieriger, da die Möglichkeit besteht, dass das 5'-Ende der cDNA für die PCR immer noch zu weit entfernt ist. Folglich wurden als zweite Option degenerierte Vorwärts-Primer zusätzlich zum definitiven Rückwärts-Primer konstruiert. Für das Design der degenerierten Primer wurden die besten zehn vollständigen Consensus-Gesamtsequenz-Treffer bezüglich der erhaltenen Sequenz verglichen, und Primer wurden aus den am stärksten konservierten Regionen dieser Sequenzen konstruiert, die stromauf der hierbei aufzulösenden Position lagen. Im Falle der degenerierten PW-3-Vorwärts-Primer 1 und 3 war der Degenerationsgrad sehr hoch. Um die Degeneration zu verringern, wurde die Base Desoxyinosin (die an A, T, G und C bindet) in der Synthese eingesetzt. Der degenerierte PW-3-Vorwärts-Primer 2 weist die geringste Degeneration auf und ist der vielversprechendste Kandidat unter den konstruierten degenerierten Primern. Außerdem wurden noch zwei Rückwärts-Primer aus der DNA-Sequenz der soeben erhaltenen Sequenz konstruiert. Der Vergleich dieser Sequenzen mit den konservierten Regionen, die für das Primer-Design ausgewählt wurden, ist nachstehend zu sehen. Definitive Rückwärts-Primer wurden ebenfalls konstruiert. Kürzere Versionen der Rückwärts1- und Rückwärts2-Primer wurden auch konstruiert, für den Fall, dass die Annealing-Temperatur der degenerierten Vorwärts-Primer niedrig war. Außerdem wurde ein dritter Rückwärts-Primer konstruiert, der ein Stück des Rückwärts-1 - und des Rückwärts-2-Primers umfasste.
degenerierter PW-3-Vorwärts-Primer 1: CANARRHTIAARYTISANAA - HRMKLEK
degenerierter PW-3- Vorwärts-Primer 2: AAYTWYATGAAYGCNCARTA - NYMNAQY
degenerierter PW-3- Vorwärts-Primer 3: TTYAARGTIRTNYTTIGAYAC - FKVILDT
definitiver PW-3-Rückwärts-Primer1: GCCAAAGGCAAATGCGAG - LAFAFG (reverses Komplement)
Pw3-Rückwärts-Primer1 kurz: GCCAAAGGCAAATGCG
definitiver PW-3-Rückwärts-Primer2: CAGGCCGAGGATACCATC - DGILGL (reverses Komplement)
Pw3-Rückwärts-Primer2kurz: CAGGCCGAGGATACC
Pw3-Rückwärts-Primer23x: CATCGAACTTGCCAAAG - FGKFDX

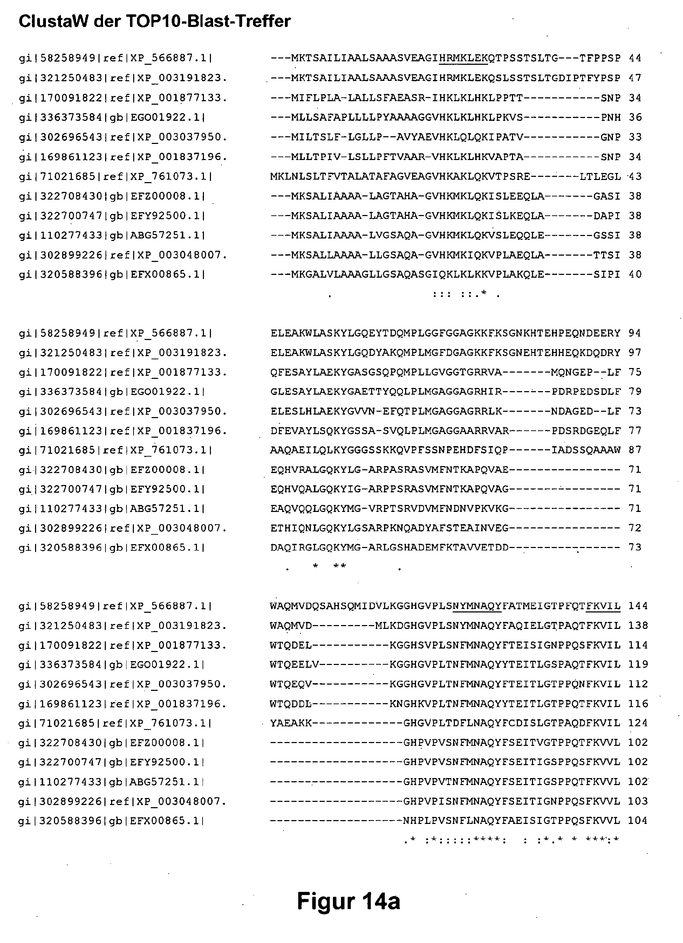

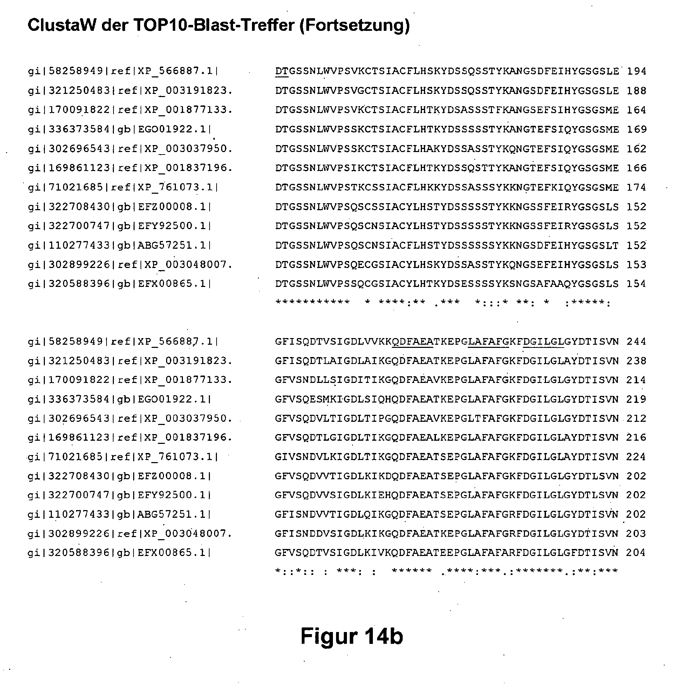

Die ClustaW-Sequenzabgleichsergebnisse der besten zehn pBLAST-Treffer sind in den Fig. 14a und 14b angeführt. Die konservierten Sequenzen, die für das Design der degenerierten Vorwärts-Primer für Primer-Walking 3 verwendet wurden, und die relative Position der definitiven Rückwärts-Primer sind markiert.

### 1.2.3.4.2 Optimierung der Temperatur- und Pufferbedingungen

Der Reaktionspuffer und die Temperatur des Primer-Annealing mussten für die PCR optimiert werden, weil degenerierte Vorwärts-Primer verwendet wurden. Unterschiedliche Kombinationen der Vorwärts- und Rückwärts-Primer wurden eingesetzt. Zur Optimierung der Bedingungen wurde ein degenerierter Vorwärts-2-Primer in Kombination mit den kurzen Rückwärts-Primern verwendet. Die für die unterschiedlichen Primerkombinationen, Annealing-Temperaturen und Puffer verwendete Terminologie ist nachstehend angeführt. Die Komponenten der unterschiedlichen PCR-Reaktionen, die durchgeführt wurden, sind in der Tabelle zusammengefasst.
* Nummerierung von Primerkombinationen
   Primerkombination-I : degenerierter PW-3-Fwd-2 und PW-3-Rev1_kurz(a)
   Primerkombination-II : degenerierter PW-3-Fwd-2 und PW-3-Rev2_kurz(a)
   Primerkombination-III : SMART IV Fwd und PW-3-Rev2 (b)
   Die PCR wurde mit der Primerkombination III durchgeführt, sodass sie als Matrize für eine Nested-Primer-PCR als nächsten Schritt eingesetzt werden konnte.
* Annealing-Temperatur
   a - 45,4 °C
   b - 59,8 °C
* Puffereinsatz
   1 - 5X HF Puffer
   2 - 5X GC Puffer
   3 - 5X HF Puffer + DMSO

**Tabelle 11: Reaktionskomponenten für die PCR unter Verwendung der unterschiedlichen Primerkombinationen für Primer-Walking 3**

| **Inhalt** | **Reaktionskomponenten für verschiedene Primerkombinationen** | | | | | | |
|---|---|---|---|---|---|---|---|
| | **I (45,4 °C) (µl)** | | | **II (45,4 °C) (µl)** | | | **III (59,8 °C) (µl)** |
| | 1 | 2 | 3 | 1 | 2 | 3 | 1 |
| Puffer (5X HF) | 10 | 10 | 10 | 10 | 10 | 10 | 10 |
| dNTPs (10mM) | 1 | 1 | 1 | 1 | 1 | 1 | 1 |
| Vorwärts-Primer (unverdünnt/1:10 verd.) | 1 | 1 | 1 | 1 | 1 | 1 | 1 |
| Rückwärts-Primer (1:10 verdünnt) | 1 | 1 | 1 | 1 | 1 | 1 | 1 |
| cDNA (350 ng/µl) | 0,5 | 0,5 | 0,5 | 0,5 | 0,5 | 0,5 | 0,5 |
| DMSO | - | - | 1,5 | - | - | 1,5 | - |
| steriles Wasser | 36 | 36 | 34,5 | 36 | 36 | 34,5 | 36 |
| DNA-Polymerase (Phusion) | 0,5 | 0,5 | 0,5 | 0,5 | 0,5 | 0,5 | 0,5 |

**Tabelle 12: PCR-Programm zur Puffer- und Annealingtemperatur-Optimierung in Primer-Walking 3**

| **Schritt** | **Temperatur (°C)** | **Dauer** | **Zyklenanzahl** |
|---|---|---|---|
| Anfangsdenaturierung | 98 | 2 min | 1 |
| Denaturierung | 98 | 30 s | |
| Annealing | T=50 | 45 s | 35 |
| | Gradient = 10 °C | | |
| | Rate = 3 °C/s | | |
| Extension | 72 | 1 min | |
| Endextension | 72 | 7 min | 1 |

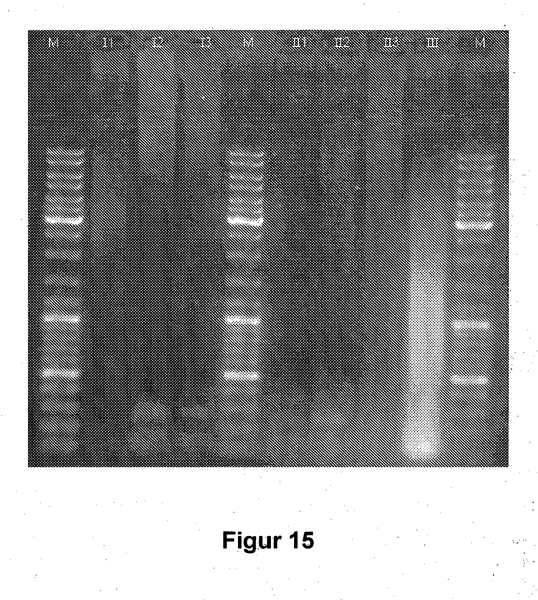

Fig. 15 ist ein Elektropherogramm der bezüglich Puffer und Annealingtemperatur optimierten PCR-Reaktionen auf 1 % Agarosegel. Die Proben (30 µl) wurden dabei jeweils auf Agarosegel analysiert. Das PCR-Produkt III wurde als Matrizen-DNA für weitere Nested-PCR-Reaktionen verwendet. Da ein PCR-Produkt beobachtet wurde, als für die Primerkombinationen I und II ein GC-Puffer verwendet wurde, nicht jedoch, als nur ein HF-Puffer verwendet wurde, wurde der GC-Puffer als bevorzugter Puffer für die Nested-PCR genommen. Die gewählte Annealing-Temperatur betrug 45,4 °C für die Nested-Primer-PCR, da bei dieser Temperatur eine Produktbildung zu beobachten war.

### 1.2.3.4.3 Nested-Primer-PCR

Das Produkt III aus der PCR-Reaktion vom Vortag wurde als Matrizen-DNA für die Nested-PCR verwendet. Hier wurden alle Primerkombinationen getestet, von denen vermutet wurde, dass sie innerhalb der Matrize liegen. Außerdem wurde der am besten für die Nested-Primer-PCR geeignete Reaktionspuffer ebenfalls bezüglich der Kombination aus degeneriertem PW-3-Vorwärts-2-Primer (Primer mit dem geringsten Degenerierungsgrad) und PW-3-Rückwärts-1-kurz-Primer getestet. Die für die Primerkombinationen verwendete Terminologie ist nachstehend angegeben. Die Komponenten der PCR-Reaktion sind in Tabelle 13 zusammengefasst.
* Primerkombinationen/Matrizen-DNA
   I - Pw3-FwPrimer1 und Pw3-RevPrimer1 kurz / PCR-Produkt III aus dem letzten Schritt
   II - Pw3-FwPrimer2 und Pw3-RevPrimer1 kurz / PCR-Produkt III aus dem letzten Schritt
   III - Pw3-FwPrimer3 und Pw3-RevPrimer1kurz / PCR-Produkt III aus dem letzten Schritt
   IV - Pw3-FwPrimer3 und Pw3-RevPrimer1kurz / PCR-Produkt II 2 aus dem letzten Schritt
   V - Pw3-FwPrimer2 und Pw3-RevPrimer23x / PCR-Produkt III aus dem letzten Schritt
* PCR-Reaktionspuffer
   1- GC-Puffer
   2- GC-Puffer mit DMSO
   3- HF-Puffer
   4- HF-Puffer mit DMSO

**Tabelle 13: Reaktionskomponenten für eine PCR unter Verwendung der unterschiedlichen Primerkombinationen für Primer-Walking 3**

| **Inhalt** | **Reaktionskomponenten für verschiedene Primerkombinationen (µl) (bei einer Annealing-Temperatur von 45,4 °C)** | | | | | | | |
|---|---|---|---|---|---|---|---|---|
| | **I1** | **II1** | **III1** | **II2** | **II3** | **II4** | **IV1** | **V1** |
| Puffer (5X 1 (oder) 2) | 10 | 10 | 10 | 10 | 10 | 10 | 10 | 10 |
| dNTPs (10mM) | 1 | 1 | 1 | 1 | 1 | 1 | 1 | 1 |
| Vorwärts-Primer (unverdünnt) | 1 | 1 | 1 | 1 | 1 | 1 | 1 | 1 |
| Rückwärts-Primer (1:10 verdünnt) | 1 | 1 | 1 | 1 | 1 | 1 | 1 | 1 |
| Matrizen-DNA | 1 | 1 | 1 | 1 | 1 | 1 | 5 | 1 |
| DMSO | - | - | - | 1,5 | - | 1,5 | - | - |
| steriles Wasser | 36 | 36 | 36 | 34,5 | 36 | 34,5 | 32 | 36 |
| DNA-Polymerase (Phusion) | 0,5 | 0,5 | 0,5 | 0,5 | 0,5 | 0,5 | 0,5 | 0,5 |

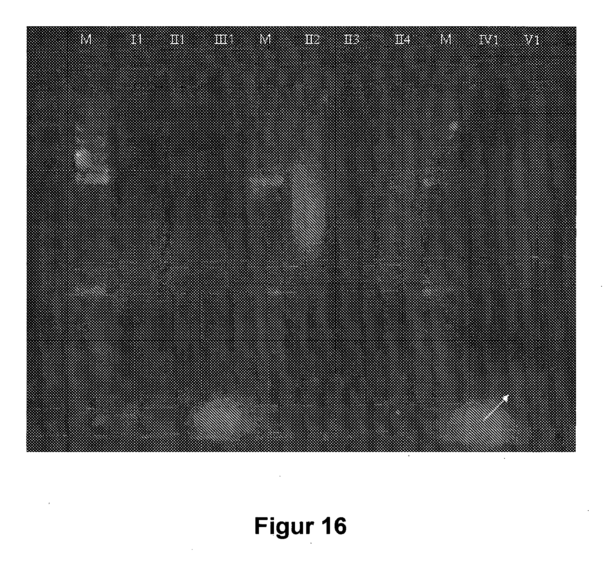

Fig. 16 zeigt ein Elektropherogramm der Nested-Primer-PCR-Reaktion auf 1 % Agarosegel. Die Bande aus Probe V1 wurde zur Extraktion von DNA, Amplifikation des PCR-Produkts durch Klonierung und Transformation und Sequenzanalyse ausgeschnitten.

### 1.2.3.4.4 Sequenzanalyse für Primer-Walking 3

Die erhaltenen DNA-Sequenzen wurden analysiert, um die Primer und somit die Sequenz der Inserts zu identifizieren. Alle Inserts führten zur selben Sequenz. Die Identität der erhaltenen Sequenzen wurde verglichen. Es zeigte sich, dass die Sequenzen vollständig ident waren.
FwPrimer 1: CANARRHTIAARYTISANAA - HRMKLEK
FwPrimer2: AAYTWYATGAAYGCNCARTA - NYMNAQY
FwPrimer3: TTYAARGTIRTNYTTIGAYAC - FKVILDT
RevPrimer1: GCCAAAGGCAAATGCGAG - LAFAFG (reverses Komplement)
RevPrimer1 short: GCCAAAGGCAAATGCG
RevPrimer2: CAGGCCGAGGATACCATC - DGILGL (reverses Komplement)
RevPrimer2short: CAGGCCGAGGATACC
RevPrimer23x: CATCGAACTTGCCAAAG - FGKFDX

>PW-3-V2-2.pJET1.2-F
>EMBOSS_001_5

### Proteinsequenz nach dem Primer-Walking-Schritt 1:

>EMBOSS_001_3

### Proteinsequenz nach dem Primer-Walking-Schritt 2:

### Proteinsequenz aus dem Primer-Walking-Schritt 3:

### Proteinsequenz nach dem Primer-Walking-Schritt 3:

Primer-Walking 3 unter Verwendung der neu konstruierten Rückwärts-Primer und der degenerierten Vorwärts-Primer war erfolgreich. Die Sequenz konnte verifiziert werden, da sie die Primersequenzen enthielt, die zuvor konstruiert worden waren. 102 weitere Aminosäurereste wurden durch Primer-Walking 3 bestimmt. Nach Primer-Walking 3 waren 319 Aminosäurereste des Proteins bestimmt. Die Proteinsequenz nach Primer-Walking 3 ist nachstehend erneut zusammenhängend angeführt:

### 1.2.3.5 Primer-Walking 4

Für Primer-Walking 4 wurden neue Rückwärts-Primer konstruiert, und die PCR wurde unter Verwendung des SMART-IV-Primers (dem Beginn jeder cDNA) als Vorwärtsprimer durchgeführt. Dabei wurden verschiedene Bedingungen bezüglich Puffer, Temperatur und Extension/Annealing-Zeiten untersucht, allerdings führten keine der getesteten Bedingungen zur Ausbildung klarer Banden, sondern es war jedesmal ein Verschmieren zu beobachten. Möglicherweise band SMART IV, der bei allen DNA-Stücken enthalten war, an mehreren Stellen und erfuhr so Extension. Aufgrund des Versagens sämtlicher Testbedingungen wurde das im Handel erhältliche SpeedUp™ Premix Kit II für DNA-Walking (Katalognr. K1503, Seegene, Südkorea) im letzten Schritt des Primer-Walkings eingesetzt.

Das Set besteht aus einem PCT-Master-Mix und einzigartigen DNA-Walking-ACP-Primern, die so konstruiert sind, dass sie unbekannte Zielstellen mit hoher Spezifität erfassen. Einer der vier ACP-Primer und der von den Erfindern selbst konstruierte targetspezifische Primer 1 (TSP1) wurden zur Amplifikation der Zielregion aus dem Templat in der ersten PCR eingesetzt. In der zweiten PCR (die ersten genesteten PCR) wurden dann der DW-ACPN-Primer und der von den Erfindern selbst konstruierte targetspezifische Primer 2 (TSP2) zur Amplifikation des Targets aus dem ersten PCT-Produkt eingesetzt. In der dritten PCR (der zweiten genesteten PCR) wurden universelle Primer und der dritte von den Erfindern selbst konstruierte targetspezifische Primer 2 (TSP3) sowie das zweite PCR-Produkt als Templat eingesetzt.

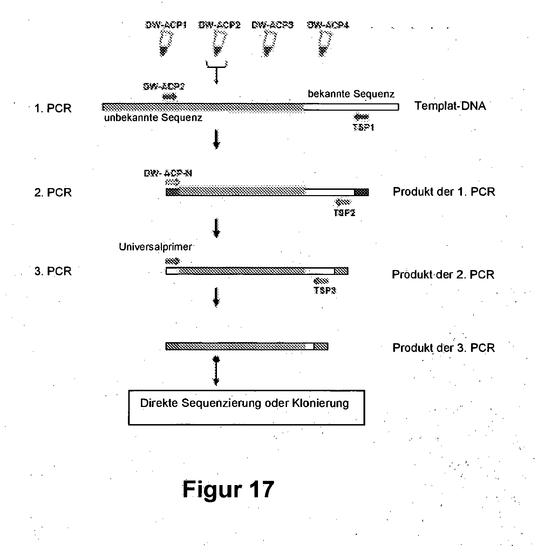

Fig. 17 zeigt diese allgemeine Vorgangsweise beim DNA-Walking unter Anwendung der ACP™-PCR-Technologie.

### 1.2.3.5.1 Design von targetspezifischen Primern

Die Anforderungen für die Konstruktion der TSPs waren die folgenden:
1. Die Primer sollten keine Haarnadel-Strukturen erzeugen.
2. Ihre 3'-Enden sollten miteinander keine Basenpaarungen eingehen.
3. Pepetitive Sequenzen oder Regionen, die Stücke desselben Nucleotids enthalten, mussten vermieden werden.
4. Überlappen der TSP-Primer war erlaubt.
5. Stromauf des TSP1-Primers sollten keine ACP-Primer-Bindungsstellen liegen (5'-AACGG; 5'-CTCGA; 5'-CTACG; 5'-ACGTG).
6. Die Primer sollten 18 bis 23 bp lang sein, zwischen 40 % und 60 % GC-Gehalt und eine Tm von 60 °C bis 65 °C aufweisen.

Beim ersten Versuch wurden drei TSP1-Primer (1.1, 1.2, 1.3) konstruiert, um mehrere davon zur Verfügung zu haben, falls der eine oder andere nicht funktionieren sollte. Die targetspezifischen Primer sind nachstehend angegeben.

| | |
|---|---|
| TSP1.3-Primer: | CCC AGT GTC CAG GAT GAC |
| TSP1.2-Primer: | ACC TTG AAC GAT TGC GGG |
| TSP1.1-Primer: | GGG AGT GCC CAA GGT GA |
| TSP2-Primer: | GAG TGC CCA AGG TGA TTT C |
| TSP3-Primer: | GGT GAT TTC AGC GAA GTA CT |

### 1.2.3.5.2 Nested-Primer-PCR

Es wurden nacheinander drei PCR-Reaktionen durchgeführt. Die erste PCR-Reaktion wurde parallel in vier verschiedenen Röhrchen unter Verwendung von Primer-Paaren durchgeführt, die Kombinationen des TSP1.1-Primers mit jeweils einem der Primer DW2-ACP1, -2, -3 und -4 waren. Um Zeit zu sparen, wurde dieselbe Vorgangsweise auch für TSP1.2 gewählt. Insgesamt erfolgte die PCR also in 8 Reaktionsröhrchen mit dem in nachstehender Tabelle 14 angegebenen Inhalt.

**Tabelle 14: Inhalt der 8 Reaktionsröhrchen für die erste PCR von Primer-Walking 4**

| **Inhalt** | | **Volumen (µl)** |
|---|---|---|
| 1. | *T. hirsuta*-cDNA-Templat (350 ng/µl) | 0,2 |
| 2. | DW2-ACP (einer von DW2-ACP1, -2, -3 und -4) (5 µM) | 2 |
| 3. | TSP1 (1.1 oder 1.2) | 1 |
| 4. | Destilliertes Wasser | 6,8 |
| 5. | 2X SeeAmp™ACP™Master Mix II | 10 |

Die PCR wurde unter Verwendung eines auf 94 °C vorgeheizten Thermozyklierers durchgeführt (Hotstart). Das PCR-Programm für die erste PCR ist in nachstehender Tabelle 15 angegeben.

**Tabelle 15: PCR-Programm für die erste PCR von Primer-Walking 4**

| **Schritt** | **Temperatur (°C)** | **Zeit** | **Zyklenanzahl** |
|---|---|---|---|
| Anfangsdenaturierung | 94 | 5 min | 1 |
| Anfangsannealing | 42 | 1 min | 1 |
| Anfangsextension | 72 | 2 min | 1 |
| Denaturierung | 94 | 30 s | |
| Annealing | 55 | 30 s | 30 |
| Extension | 72 | 100 s | |
| Endextension | 72 | 7 min | 1 |

Die erhaltenen PCR-Produkte wurden unter Verwendung eines PCR-Reinigungssets (QIAGEN, Katalognr. 28106) gereiningt. Die gereinigten Produkte der 8 Röhrchen wurden als Templat-DNA für die zweite PCR eingesetzt. Der Inhalt der zweiten PCR-Reaktionsröhrchen ist in Tabelle 16 angegeben.

**Tabelle 16: Inhalt der 8 Reaktionsröhrchen für die zweite PCR von Primer-Walking 4**

| **Inhalt** | | **Volumen (µl)** |
|---|---|---|
| 1. | gereinigtes Produkt der ersten PCR | 3 |
| 2. | DW2-ACPN (5 µM) | 2 |
| 3. | TSP2 | 1 |
| 4. | Destilliertes Wasser | 4 |
| 5. | 2X SeeAmp™ACP™Master Mix II | 10 |

Für die Durchführung der PCR wurde der Thermozyklierer erneut auf 94 °C vorgeheizt. Das PCR-Programm ist in 17 angegeben.

**Tabelle 17: PCR-Programm für die zweite PCR von Primer-Walking 4**

| **Schritt** | **Temperatur (°C)** | **Zeit** | **Zyklenanzahl** |
|---|---|---|---|
| Anfangsdenaturierung | 94 | 3 min | 1 |
| Denaturierung | 94 | 30 s | |
| Annealing | 60 | 30 s | 35 |
| Extension | 72 | 100 s | |
| Endextension | 72 | 7 min | 1 |

Die dritte PCR, d.h. die zweite Nested-PCR, wurde unter Verwendung der PCR-Produkte aus dem zweiten Schritt als DNA-Template und von Universalprimern als Primerpaare durchgeführt. Der Inhalt der PCR-Röhrchen ist in Tabelle 18 und das PCR-Programm in Tabelle 19 angegeben. Auch in diesem Fall wurde der Thermozyklierer auf 94 °C vorgeheizt.

**Tabelle 18: Inhalt der Reaktionsröhrchen für die dritte PCR von Primer-Walking 4**

| **Inhalt** | | **Volumen (µl)** |
|---|---|---|
| 1. | gereinigtes Produkt der zweiten PCR | 2 |
| 2. | UniP2 (5 µM) | 1 |
| 3. | TSP3 | 1 |
| 4. | Destilliertes Wasser | 6 |
| 5. | 2X SeeAmp™ACP™Master Mix II | 10 |

**Tabelle 19: PCR-Programm für die dritte PCR von Primer-Walking 4**

| **Schritt** | **Temperatur (°C)** | **Zeit** | **Zyklenanzahl** |
|---|---|---|---|
| Anfangsdenaturierung | 94 | 3 min | 1 |
| Denaturierung | 94 | 30 s | |
| Annealing | 60 | 30 s | 30 |
| Extension | 72 | 100 s | |
| Endextension | 72 | 7 min | 1 |

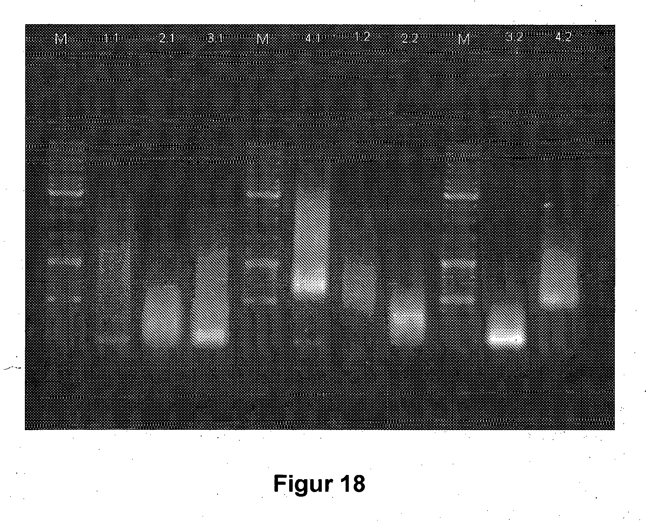

Schließlich wurden 10-µl-Proben auf Agarosegel (1 %) analysiert. Die Ergebnisse werden in Fig. 18 gezeigt. Die Banden in Spur 3.1 (etwa 400 bp), Spur 2.2 (etwa 300 bp) und Spur 4.2 (etwa 500 bp) wurden aus dem Gel ausgeschnitten, und die DNA wurde unter Verwendung des Gel-Extraktionssets von QIAGEN extrahiert. Die ausgeschnittenen PCR-Produkte hatten überstehende Enden, weswegen diese vor dem Klonieren in den pJET1.2-Vektor (stumpfendige Ligation) mit einem Abstumpfungsenzym abgestumpft werden mussten. Das Abstumpfungsenzym stand im CLONEJET™-PCT-Klonierungsset zur Verfügung. Das Ligationsgemisch wurde danach in kompetente TOP10-Zellen transformiert. Die Transformanten wurden herausgepickt, damit Übernachtkulturen angesetzt, die Plasmide isoliert und die DNA-Sequenzen analysiert.

### 1.2.3.5.3 Analyse der DNA-Sequenzen

Die Sequenz der das Insert enthaltenden Plasmide wurde analysiert. Eines der Produkte enthielt tatsächlich den Teil des Gens von Interesse. In der Sequenz konnten der TSP3-Primer und der Universalprimer identifiziert werden. Die Translation der DNA-Sequenz umfasste den bereits bekannten Teil des Gens von Interesse, was bestätigte, dass er im Gen enthalten war. Der Anfang des Gens war nun erreicht. Die erzielten DNA-Sequenzierungsergebnisse sind nachstehend angeführt.
>Seegene-2-Bande-4-1-1-3-Vorwärts.pJET1.2-F Translation:
>EMBOSS_001_3 >Seegene-2-Bande-4-1-1-3-Rückwärts.pJET1.2-R
Translation:
>EMBOSS_001_4
   1) Universalprimer:
      GAGTTTAGGTCCAGCGTCCGT
      Reverses Komplement:
      ACGGACGCTGGACCTAAACTC
   2) PW-4 TSP-3:
      GGTGATTTCAGCGAAGTACT
      Reverses Komplement:
      AGTACTTCGCTGAAATCACC

Die im Primer-Walking-Schritt 4 (mit dem Seegene-Set) erhaltene Proteinsequenz ist nachstehend angegeben.

### Proteinsequenz nach dem Primer-Walking-Schritt 4:

Nach dem Primer-Walking 4, bei dem 100 Aminosäurereste erhalten wurden, war die Peptidsequenz des Gens von Interesse vollständig. Das gesamte Protein, beginnend mit einem Startcodon und vor dem Stopcodon endend, bestand somit aus 412 Aminosäuren.

Die vollständige Aminosäuresequenz wurde als Seq.-ID Nr. 1 bezeichnet und ist nachstehend noch einmal zusammenhängend im Einbuchstabencode angegeben:
Seq.-ID Nr. 1: MILSRFAPLALLPFVAADGVHKLKLTKLPPATSNPLLESAYLAEKYGGGSQMPLSAGIGRNVRVSRPTV KDGEELFWTQDEFSTEGGHNVPLSNFMNAQYFAEITLGTPPQSFKVILDTGSSNLWVPSTKCTSIACF LHAKYDSTASSTYKANGSEFSIQYGSGSMEGFVSQDVLTIGDITIKNQDFAEATKEPGLAFAFGKFDGI LGLGYDTISVNHITPPFYQMMNQKLVDSPVFSFRLGSSEEDGGEAIFGGVDETAYSGKIEYVPVRRKA YWEVELESIKLGDDELELDNTGAAIDTGTSLIALPSDLAEMLNVQIGAKKSWNGQYTVDCAKVPTLPDL TFYFSGKPYTLKGTDYVLEVQGTCMSSFTGIDINLPGGGALWIIGDVFLRKYYTVYDHGRDAVGFALA K*

Die entsprechende Sequenz im Dreibuchstabencode findet sich im beiliegenden Sequenzprotokoll.

### 1.2.3.6 Ableitung der vollständigen Gensequenz

Zur Ableitung der vollständigen Gensequenz wurden die aus einer anfänglichen PCR und den verschiedenen anschließenden Primer-Walking-Schritten erhaltenen Genabschnitte zusammengesetzt. Diese vollständige Gensequenz wurde Seq.-ID Nr. 3 genannt und ist nachstehend angegeben:
Seq.-ID Nr. 3:

### 1.2.3.7 Isolierung des vollständigen Gens aus cDNA und genomischer DNA

Für die Isolierung des vollständigen Gens aus cDNA und genomischer DNA wurden neue Primer konstruiert. Das Gen musste auch aus genomischer DNA isoliert werden, um die beiden Sequenzen vergleichen und feststellen zu können, ob es bei der reversen Transkription zu etwaigen Mutationen gekommen war. Die konstruierten Primer umfassten eine Ndel-Restriktibrisstelle am Vorwärtsprimer und eine Xhol-Restriktionsstelle am Rückwärtsprimer. Die Primersequenzen sind in obiger Seq.-ID Nr. 2 markiert.
Vorwärts-Primer:
   AAT TAC *ATA TGA TAC TCT CCA GAT TCG CCC CC* (Ndel-Stelle)
Rückwärts-Primer:
   AATTCTC GAG TC*A CTT GGC AAG AGC GAA GCC* (Xhol-Stelle)

Es wurden zwei PCR-Reaktionen durchgeführt, eine mit cDNA von *T.hirsuta* als Templat und die andere mit genomischer DNA von *T.hirsuta* als Templat. Zur Isolierung der genomischen DNA aus dem Pilz wurde das peqGOLD Tissue DNA Mini Kit (Bestellnr. 12-3396-00) eingesetzt. Die beiliegende Arbeitsvorschrift des Herstellers wurde genau befolgt, wobei aus 40 mg lyophilisierter Zellen 35 µg genomische DNA isoliert wurden. Die Zusammensetzung der Reaktionsgemische für die PCR ist in Tabelle 20 und das PCR-Programm in Tabelle 21 angegeben.

**Tabelle 20: PCR-Reaktionsgemische zum Erhalt des vollständigen Gens aus cDNA bzw. genomischer DNA**

| **Inhalt** | **Volumen (µl)** | |
|---|---|---|
| | **cDNA als Templat** | **genomische DNA als Templat** |
| Phusion-Puffer 5X HF | 10 | 10 |
| dNTPs (10 mM) | 1 | 1 |
| Vorwärtsprimer (1:10 verdünnt) | 1 | 1 |
| Rückwärtsprimer (1:10 verdünnt) | 1 | 1 |
| Templat-DNA (350 ng/µl) | 0.5 | 0.5 |
| steriles Wasser | 36 | 36 |
| DNA-Polymerase (Phusion) | 0.5 | 0.5 |

**Tabelle 21: PCR-Programm zur Amplifikation des Gens aus cDNA bzw. genomischer DNA**

| **Schritt** | **Temperatur (°C)** | **Zeit** | **Zyklenanzahl** |
|---|---|---|---|
| Anfangsdenaturierung | 98 | 3 min | 1 |
| Denaturierung | 98 | 30 s | |
| Annealing | 65 | 30 s | 40 |
| Extension | 72 | 100s | |
| Endextension | 72 | 7 min | 1 |

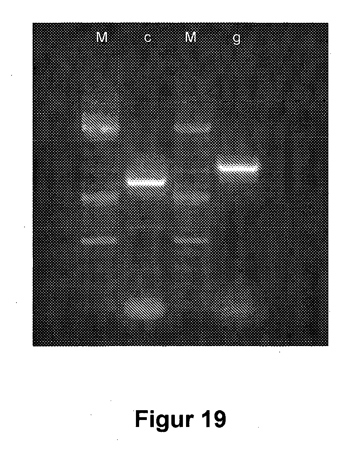

Nach der PCR wurden die Proben auf Agarosegel (1 %) neben einem Marker analysiert. Das Gen konnte sowohl aus dem cDNA-Templat als auch aus genomischer DNA als Templat amplifiziert werden. Die Analysenergebnisse werden in Fig. 19 gezeigt, wobei "c" für cDNA und "g" für genomische DNA als Templat stehen.

### 1.2.3.8 Verifizierung der erhaltenen Gensequenz

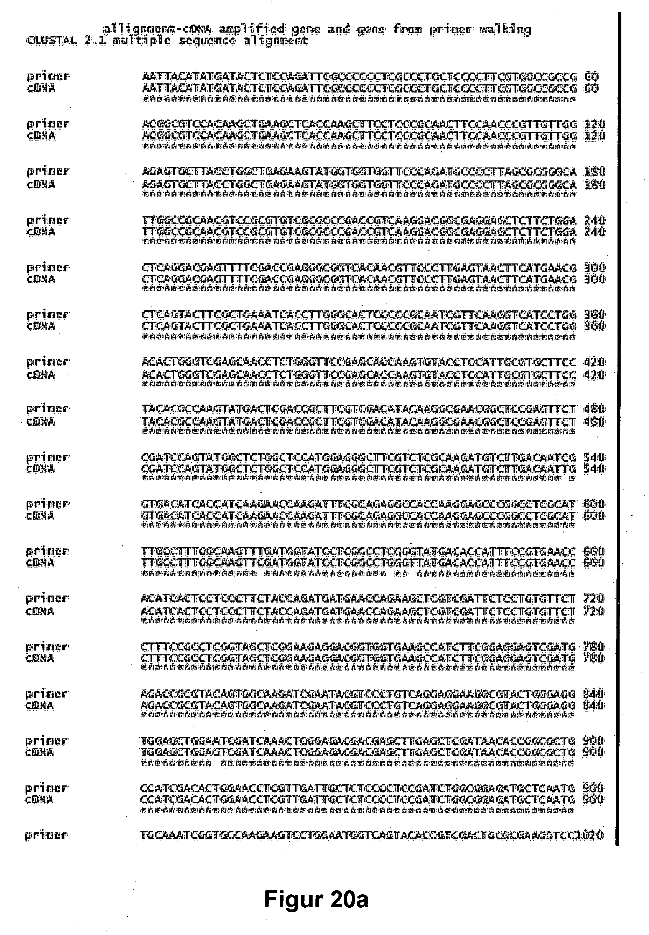

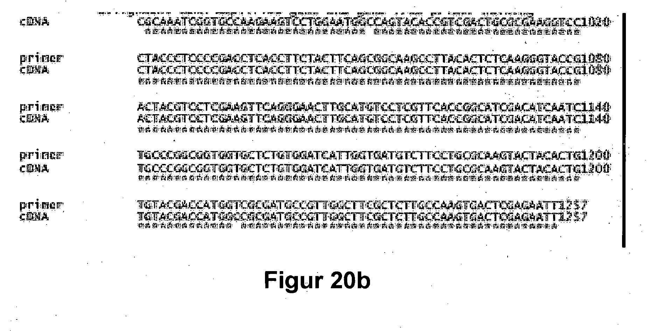

Die beiden Banden wurden aus dem Gel ausgeschnitten und die DNA daraus eluiert. Die Gene wurden in die pJET1.2-Vektoren (CLONEJET™, stumpfer Vektor) ligiert und in *E.coli*-TOP10-Zellen transformiert. Mit drei herausgepickten Kolonien wurden Übernachtkulturen angesetzt, und die Proben wurden sequenziert, um die Gensequenz zu verifizieren. Die Sequenzierungsergebnisse zeigten, dass sieben Basenpaar-Unterschiede zwischen dem isolierten vollständigen Gen und der durch Zusammensetzen der Stücke aus den vier Primer-Walking-Schritten erhaltenen Gensequenz existieren. Der Sequenzvergleich zwischen den beiden Sequenzen mittels der Clustal 2.1-Software zum Mehrsequenz-Abgleich ist in Fig. 20 angegeben.

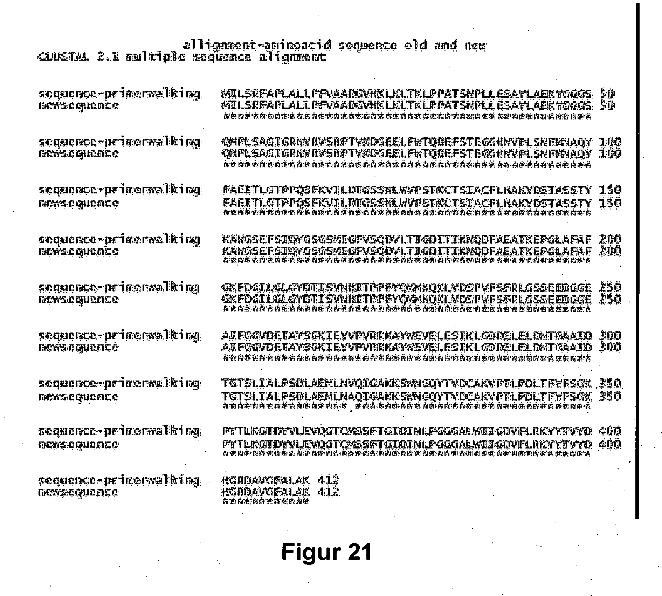

Diese Unterschiede führten allerdings lediglich zu einer einzigen Aminosäure-Differenz an Position 318, wo ein Valin anstelle von Alanin liegt. Fig. 21 zeigt einen Vergleich der Aminosäuresequenzen mittels der Clustal 2.1-Software. Der Unterschied in den Aminosäuren ist an dem fehlenden Sternchen an Position 318 zu erkennen. Die so ermittelte tatsächliche Aminosäuresequenz des isolierten Enzyms wurde als Seq.-ID Nr. 2 und die dafür kodierende Sequenz als Seq.-ID Nr. 4 bezeichnet. Beide sind im beigefügten Sequenzprotokoll angegeben.

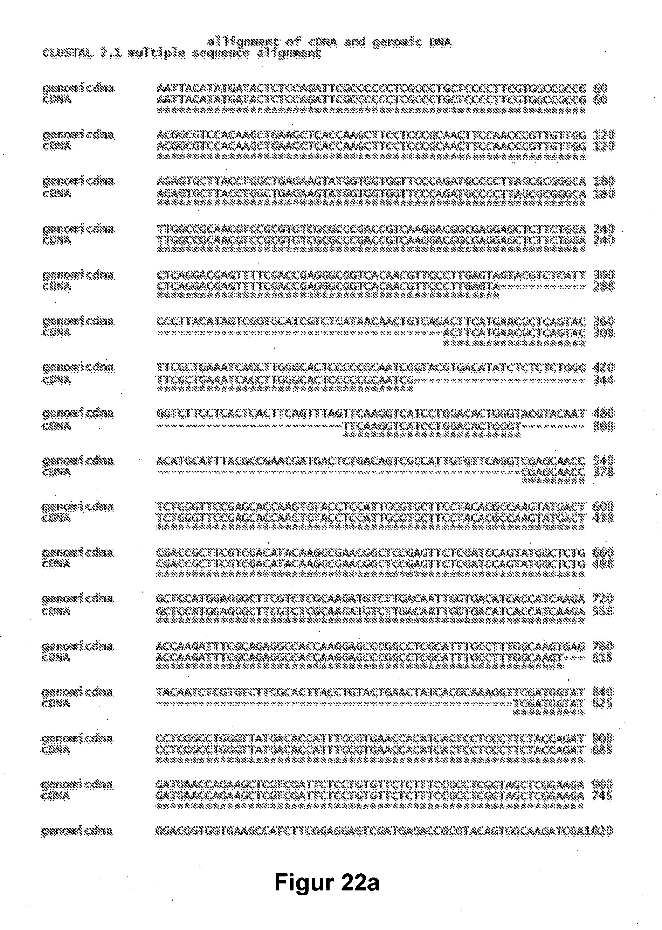

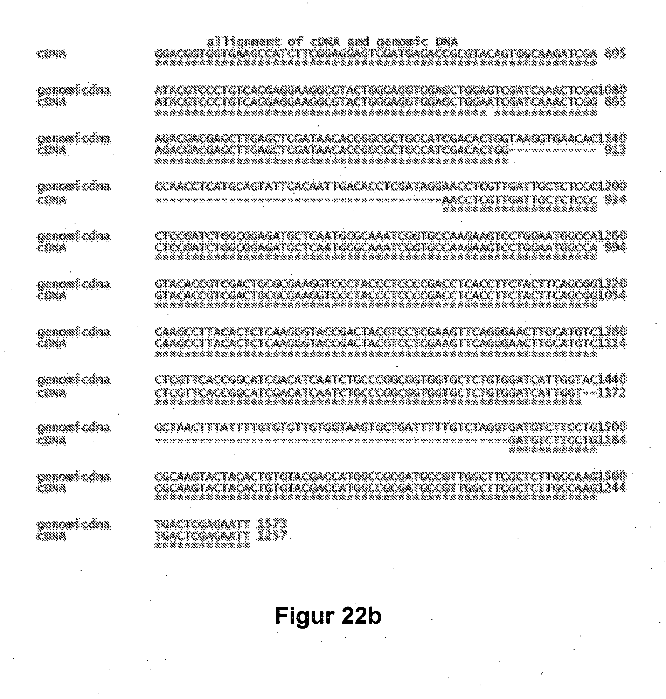

Außerdem zeigte das Überlappen der aus genomischer DNA als Templat erhaltenen Gensequenz mit der aus dem cDNA-Templat erhaltenen Gensequenz die Gegenwart von sechs im Gen verteilten Infron-Sequenzen, wie dies in dem in Fig. 22 dargestellten Clustal 2.1-Sequenzabgleich gut zu erkennen ist.

### 1.2.3.9 Ligation des Gens in den pET-21(a)-Vektor

In der Folge wurde ein Teil des aus dem cDNA-Templat erhaltenen Gens dreier Kolonien mit Ndel und Xhol aus dem pJET1.2-Vektor gespalten. Das Reaktionsgemisch für diesen Restriktionsverdau-Schritt ist in nachstehender Tabelle 22 angegeben.

**Tabelle 22: Reaktionsgemisch für den Restriktionsverdau des Gens aus dem pJET1.2-Vektor**

| **Komponente** | **Menge (µl)** |
|---|---|
| Wasser (nucleasefrei) | 13 |
| Fast Digest^{®} Green-Puffer (Fermentas) | 7,7 |
| MiniPrep-Probe (16 ng/µl) | 8 (50 ng) |
| Fast Digest^{®} Xho I | 1 |
| Fast Digest^{®} Nde I | 1 |

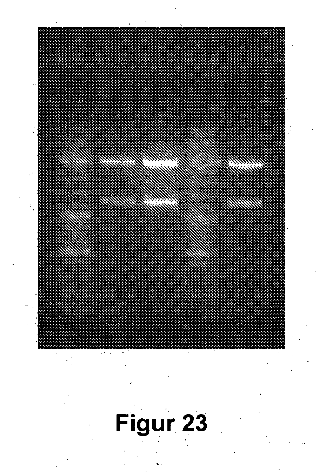

Die Proben wurden danach auf Agarosegel analysiert, und die Ergebnisse sind in Fig. 23 dargestellt.

Das restringierte Gen wurde dann in den zuvor mit demselben Paar Restriktionsenzyme gespaltenen Vektor pET-21 (a) ligiert. Die Zusammensetzung des Ligationsgemischs ist in Tabelle 23 angeführt. Der Vektor wurde anschließend in *E.coli*-XL1-Blue-Zellen transformiert, um den das Gen enthaltenden Vektor zu amplifizieren.

**Tabelle 23: Subklonieren des mit NdeI und XhoI gespaltenen Gens in pET-21(a)**

| **Komponente** | **Menge (µl)** |
|---|---|
| T4 Ligationspuffer (10X) (Fermentas) | 2 |
| Gen aus dem cDNA-Templat (12,8 ng/µl) | 4,5 |
| pET-21 a(+)-Plasmidvektor (17,8 ng/µl) | 2,8 |
| steriles Wasser | 1,3 |
| T4-DNA-Ligase (Fermentas) | 1 |

Das rekombinante Plasmid wurde danach in *E.coli*-BL(21).pTf16-Zellen transformiert. Einige Transformantenkolonien wurden herausgepickt und Übernachtkulturen davon angesetzt. Diese wurden als Keime für größere Kulturen verwendet. Die Expression des Enzyms wurde durch Zusatz von IPTG induziert. Das resultierende Zellpellet wurde in Bis-Tris-Puffer (pH 6, 50 mM) resuspendiert, ultrabeschallt, um die Zellen aufzubrechen, und zentrifugiert, um die lösliche Proteinfraktion PF1 abzutrennen, die in der Folge auf ihre katalytische Aktivität bei der Alkenspaltung untersucht wurde.

Die Fraktion PF1 wurde hierzu in je zwei Ansätzen unter Standard-Reaktionsbedingungen (6 mM *t*-Anethol als Substrat; 0,4 mM Mn(III)-acetat; 2 bar Sauerstoffdruck; Schütteln bei 170 U/min; Raumtemperatur; 36 Stunden) auf ihre Alkenspaltungsaktivität untersucht. Es wurden Umsätze des Substrats zum entsprechenden Produkt der Alkenspaltung, d.h. *p*-Anisaldehyd, von durchschnittlich 38 % festgestellt.

### 1.2.3.10 Rekombinante Produktion des Gens

Zur Expression mit His-Markierung wurde das Gen erneut in den pET-21 (a)-Vektor ligiert und in *E.coli*-BL21 Codon Plus RP (DE3) transformiert. Die Zellen wurden in LB-Medium (250 ml in 1-I-Schüttelkolben) mit Ampicillin (100 µg/l) bei 37 °C und 120 U/min kultiviert, bis die OD₆₀₀ einen Wert von 0,6 erreichte. Nach Induktion mit IPTG (Isopropyl-β-thiogalactosid; 0,3 mM) wurde das Reaktionsgemisch bei 20 °C 16 Stunden lang inkubiert. Anschließend wurden die Zellen zentrifugiert (8000 U/min, 4 °C, 20 min), das Zellpellet in Bis-Tris-Puffer (pH 6, 50 mM) resuspendiert und gefriergetrocknet. Für die anschließenden Katalyse-Reaktion wurden zellfreie Extrakte verwendet, wofür die Zellen in Puffer resuspendiert und mittels Ultraschall im Eis aufgeschlossen wurden (30 % Amplitude; 1 s Puls an, 4 s Puls aus; 2 min 30 s).

Von diesem Ansatz wurde erneut die lösliche Fraktion, PF2, entsprechend Standard-Protokollen gereinigt und dann das gereinigte Enzym auf dieselbe Weise wie oben für PF1 beschrieben auf seine Aktivität bei der Alkenspaltung getestet. Dabei konnte ein Umsatz zu *p*-Anisaldehyd von durchschnittlich 67 % erzielt werden.

Somit ist es den Erfindern gelungen, ein neues, als Katalysator bei der Alkenspaltung wirksames Enzym aus *Trametes hirsuta* zu isolieren und in der Folge seine Struktur und Aminosäuresequenz vollständig aufzuklären (Seq.-ID Nr. 1) und zu verifizieren (Seq.-ID Nr. 2). Die katalytische Wirksamkeit des Enzyms lässt sich durch Zusatz von Mn³⁺-Ionen beträchtlich steigern, was nahe legt, dass Mangan(III) einen Cofaktor für das Enyzm darstellt. Weiters wurde die Sequenz des für das Enzym kodierenden Gens (Seq.-ID Nr. 3) bestimmt, wonach das Gen ebenfalls aus dem Pilzstamm isoliert und seine Sequenz bestätigt werden konnte (Seq.-ID Nr. 4). Mit diesem Enzym stellt die vorliegende Erfindung nun einen hochwirksamen Katalysator für die biokatalysierte Spaltung von Alkenen bereit.

### Hinterlegung von biologischem Material

Gemäß den Bestimmungen des Budapester Vertrags über die internationale Anerkennung der Hinterlegung von Mikroorganismen für die Zwecke von Patentverfahren wurde am 11. April 2012 eine lebensfähige Probe des Trameten-Stamms *Trametes hirsuta* FCC 047 bei der National Collection of Agricultural and Industrial Microorganisms (NCAIM) an der Corvinus Universität in Budapest Somlói út 14-16, 1118, Ungarn, hinterlegt. Dem Stamm wurde die Zugriffsnummer NCAIM (P) F 001404 zugeteilt.

## Patentansprüche

1. Enzym, das die Aminosäuresequenz von Seq.-ID Nr. 1 oder Seq.-ID Nr. 2 aufweist oder umfasst.

2. Verwendung eines Enzyms nach Anspruch 1 oder eines Enzyms mit einer zu zumindest 80 % mit Seq.-ID Nr. 1 oder 2 identischen Aminosäuresequenz als Katalysator bei der Spaltung von Vinylaromaten mit Sauerstoff.

3. Verwendung nach Anspruch 2, **dadurch gekennzeichnet, dass** das Enzym eine Aminosäuresequenz mit zumindest 90 %, vorzugsweise zumindest 95 %, noch bevorzugter zumindest 99 %, Identität mit Seq.-ID Nr. 1 oder 2 aufweist.

4. Verwendung nach Anspruch 3, **dadurch gekennzeichnet, dass** das Enzym eine Aminosäuresequenz mit zumindest 95 % Identität mit Seq.-ID Nr. 1 oder 2 aufweist.

5. Verwendung nach Anspruch 4, **dadurch gekennzeichnet, dass** das Enzym eine Aminosäuresequenz mit zumindest 99 % Identität mit Seq.-ID Nr. 1 oder 2 aufweist.

6. Verwendung nach einem der Ansprüche 2 bis 5, **dadurch gekennzeichnet, dass** die Spaltung in Gegenwart von Mn³⁺-Ionen durchgeführt wird.

7. Verfahren zur Herstellung eines Enzyms nach Anspruch 1, **dadurch gekennzeichnet, dass** eine Kultur von *Trametes hirsuta* kultiviert wird und das Enzym aus einem zellfreien Extrakt der Kultur gewonnen wird.

8. Verfahren nach Anspruch 7, **dadurch gekennzeichnet, dass** eine Kultur von *Trametes hirsuta* G FCC 047 kultiviert wird.

9. Verfahren nach Anspruch 7 oder 8, **dadurch gekennzeichnet, dass** die Gewinnung aus dem zellfreien Extrakt mittels einer Kombination aus hydrophober Wechselwirkungschromatographie und Anionenaustauschchromatographie durchgeführt wird.

10. Verfahren nach Anspruch 9, **dadurch gekennzeichnet, dass** zur Gewinnung des Enzyms hydrophobe Wechselwirkungschromatographie, Anionenaustauschchromatographie und erneut hydrophobe Wechselwirkungschromatographie in dieser Reihenfolge durchgeführt werden.

11. Verfahren zur rekombinanten Produktion eines Enzyms nach Anspruch 1, **dadurch gekennzeichnet, dass** eine für das Enyzm kodierende Nucleinsäure mit einer Nucleotidsequenz gemäß Seq.-ID Nr. 3 bzw. Seq.-ID Nr. 4 in zur Expression der Nucleinsäure geeignete Zellen transformiert wird, die Zellen unter für die Expression der Nucleinsäure geeigneten Bedingungen inkubiert werden und das Enzym aus der Kulturbrühe isoliert wird.

12. Verfahren nach Anspruch 11, **dadurch gekennzeichnet, dass** als zur Expression der Nucleinsäure geeignete Zellen *E. coli-* oder *Pichia pastoris*-Zellen eingesetzt werden.

13. Verfahren nach Anspruch 11 oder 12, **dadurch gekennzeichnet, dass** die Nucleinsäure mit einer Nucleotidsequenz gemäß Seq.-ID Nr. 3 bzw. Seq.-ID Nr. 4 zunächst in einen Vektor ligiert wird, mit dem die Zellen anschließend transformiert werden.

14. Verfahren nach Anspruch 13, **dadurch gekennzeichnet, dass** als Vektor ein Plasmidvektor eingesetzt wird.

## Claims

1. An enzyme having or comprising the amino acid sequence of SEQ ID No. 1 or 2.

2. A use of the enzyme according to claim 1, or an enzyme having at least 80% amino acid sequence identity with SEQ ID No. 1 or 2, as a catalyst for the oxidative cleaving of vinyl aromatic compounds.

3. The use according to claim 2, **characterized in that** the enzyme has an amino acid sequence having at least 90%, preferably at least 95%, more preferably 99%, sequence identity with SEQ ID No. 1 or 2.

4. The use according to claim 3, **characterized in that** the enzyme has an amino acid sequence having at least 95% identity with SEQ ID No. 1 or 2.

5. The use according to claim 4, **characterized in that** the enzyme has an amino acid sequence having at least 99% identity with SEQ ID No. 1 or 2.

6. The use according to any one of the claims 2 to 5, **characterized in that** the cleaving is carried out in the presence of Mn³⁺ ions.

7. A method for producing the enzyme according to claim 1, **characterized in that** a culture of *Trametes hirsuta* is cultivated and the enzyme is obtained from a cell-free extract of said culture.

8. The method according to claim 7, **characterized in that** a culture of *Trametes hirsuta* G FCC 047 is cultivated.

9. The method according to claim 7 or 8, **characterized in that** the enzyme is obtained from said cell-free extract by means of a combination of hydrophobic interaction chromatography and anion exchange chromatography.

10. The method according to claim 9, **characterized in that** hydrophobic interaction chromatography, anion exchange chromatography and another hydrophobic interaction chromatography are carried out, in this sequence, to obtain the enzyme.

11. A method for recombinantly producing the enzyme according to claim 1, **characterized in that** a nucleic acid encoding said enzyme and having a nucleotide sequence according to SEQ ID No. 3 or 4 is transformed into cells suitable for expressing said nucleic acid, said cells are incubated under conditions suitable for expressing said nucleic acid, and said enzyme is isolated from the culture broth.

12. The method according to claim 11, **characterized in that** the cells suitable for expressing said nucleic acid are *E*. *coli* or *Pichia pastoris* cells.

13. The method according to claim 11 or 12, **characterized in that** said nucleic acid having a nucleotide sequence according to SEQ ID No. 3 or 4 is ligated into a vector at first, said vector being then used for transforming said cells.

14. The method according to claim 13, **characterized in that** a plasmid vector is used as said vector.

## Revendications

1. Enzyme ayant ou comprenant la séquence d'acides aminés indiquée dans SEQ ID no. 1 ou 2.

2. Utilisation d'un enzyme selon la revendication 1 ou d'un enzyme ayant au moins 80% identité de séquence avec SEQ ID no. 1 ou 2 comme catalyseur pour le clivage oxydatif de composés aromatiques vinyliques.

3. Utilisation selon la revendication 2, **caractérisée en ce que** ledit enzyme a une séquence d'acides aminés avec une identité de séquence avec SEQ ID no. 1 ou 2 d'au moins 90%, de préférence d'au moins 95% et plus préférentiellement d'au moins 99%.

4. Utilisation selon la revendication 3, **caractérisée en ce que** ledit enzyme a une séquence d'acides aminés avec une identité de séquence avec SEQ ID no. 1 ou 2 d'au moins 95%.

5. Utilisation selon la revendication 4, **caractérisée en ce que** ledit enzyme a une séquence d'acides aminés avec une identité de séquence avec SEQ ID no. 1 ou 2 d'au moins 99%.

6. Utilisation selon une des revendications 2 à 5, **caractérisée en ce que** le clivage a lieu en présence d'ions Mn³⁺.

7. Procédé pour produire un enzyme selon la revendication 1, **caractérisé en ce qu'**une culture de *Trametes hirsuta* est cultivée et que l'enzyme est obtenu d'un extrait sans cellules de ladite culture.

8. Procédé selon la revendication 8, **caractérisé en ce qu'**une culture de *Trametes hirsuta* G FCC 047 est cultivée.

9. Procédé selon la revendication 7 ou 8, **caractérisé en ce que** l'enzyme est obtenu dudit extrait sans cellules par une combinaison d'une chromatographie d'interactions hydrophobes et d'une chromatographie d'échange d'anions.

10. Procédé selon la revendication 9, **caractérisé en ce qu'**une chromatographie d'interactions hydrophobes, une chromatographie d'échange d'anions et une autre chromatographie d'interactions hydrophobes sont effectuées dans cet ordre pour obtenir ledit enzyme.

11. Procédé pour produire un enzyme selon la revendication 1 par recombinaison, **caractérisé en ce qu'**un acide nucléique encodant ledit enzyme et ayant la séquence de nucléotides selon SEQ ID no. 3 ou 4 est transformé dans des cellules appropriées pour l'expression dudit acide nucléique, lesdites cellules étant incubées dans des conditions appropriées pour l'expression de l'acide nucléique, et que ledit enzyme est isolé du bouillon de culture.

12. Procédé selon la revendication 11, **caractérisé en ce que** les cellules appropriées pour l'expression dudit acide nucléique sont des cellules d'*E*. *coli* ou de *Pichia pastoris.*

13. Procédé selon la revendication 11 ou 12, **caractérisé en ce que** ledit acide nucléique ayant une séquence de nucléotides selon SEQ ID no 3 ou 4 est ligaturé dans un vecteur qui est ensuite utilisé pour transformer lesdites cellules.

14. Procédé selon la revendication 13, **caractérisé en ce qu'**un vecteur plasmidique est utilisé comme vecteur.


### SEQUENZPROTOKOLL

<110> Universität Graz
<120> Enzymatische Alkenspaltung
<130> PCT238
<150> AT A 1221/2011
   <151> 2011-08-26
<160> 4
<210> 1
   <211> 412
   <212> PRT
   <213> *Trametes hirsuta*
<220>
   <221>
   <222> (1) ... (412)
   <223> mittels Primer-Walking bestimmte Aminosäuresequenz
<400> 1
   Met Ile Leu Ser Arg Phe Ala Pro Leu Ala Leu Leu Pro
   1               5                   10
   Phe Val Ala Ala Asp Gly Val His Lys Leu Lys Leu Thr
       15                  20                  25
   Lys Leu Pro Pro Ala Thr Ser Asn Pro Leu Leu Glu Ser
               30                  35
   Ala Tyr Leu Ala Glu Lys Tyr Gly Gly Gly Ser Gln Met
   40                  45                  50
   Pro Leu Ser Ala Gly Ile Gly Arg Asn Val Arg Val Ser
           55                  60                  65
   Arg Pro Thr Val Lys Asp Gly Glu Glu Leu Phe Trp Thr
                   70                  75
   Gln Asp Glu Phe Ser Thr Glu Gly Gly His Asn Val Pro
       80                  85                  90
   Leu Ser Asn Phe Met Asn Ala Gln Tyr Phe Ala Glu Ile
               95                  100
   Thr Leu Gly Thr Pro Pro Gln Ser Phe Lys Val Ile Leu
   105                 110                 115
   Asp Thr Gly Ser Ser Asn Leu Trp Val Pro Ser Thr Lys
           120                 125                 130
   Cys Thr Ser Ile Ala Cys Phe Leu His Ala Lys Tyr Asp
                   135                 140
   Ser Thr Ala Ser Ser Thr Tyr Lys Ala Asn Gly Ser Glu
       145                 150                 155
   Phe Ser Ile Gln Tyr Gly Ser Gly Ser Met Glu Gly Phe
               160                 165
   Val Ser Gln Asp Val Leu Thr Ile Gly Asp Ile Thr Ile
   170                 175                 180
   Lys Asn Gln Asp Phe Ala Glu Ala Thr Lys Glu Pro Gly
           185                 190                 195
   Leu Ala Phe Ala Phe Gly Lys Phe Asp Gly Ile Leu Gly
                   200                 205
   Leu Gly Tyr Asp Thr Ile Ser Val Asn His Ile Thr Pro
       210                 215                 220
   Pro Phe Tyr Gln Met Met Asn Gln Lys Leu Val Asp Ser
               225                 230
   Pro Val Phe Ser Phe Arg Leu Gly Ser Ser Glu Glu Asp
   235                 240                 245
   Gly Gly Glu Ala Ile Phe Gly Gly Val Asp Glu Thr Ala
           250                 255                 260
   Tyr Ser Gly Lys Ile Glu Tyr Val Pro Val Arg Arg Lys
                   265                 270
   Ala Tyr Trp Glu Val Glu Leu Glu Ser Ile Lys Leu Gly
       275                 280                 285
   Asp Asp Glu Leu Glu Leu Asp Asn Thr Gly Ala Ala Ile
               290                 295
   Asp Thr Gly Thr Ser Leu Ile Ala Leu Pro Ser Asp Leu
   300                 305                 310
   Ala Glu Met Leu Asn Val Gln Ile Gly Ala Lys Lys Ser
           315                 320                 325
   Trp Asn Gly Gln Tyr Thr Val Asp Cys Ala Lys Val Pro
                   330                 335
   Thr Leu Pro Asp Leu Thr Phe Tyr Phe Ser Gly Lys Pro
       340                 345                 350
   Tyr Thr Leu Lys Gly Thr Asp Tyr Val Leu Glu Val Gln
               355                 360
   Gly Thr Cys Met Ser Ser Phe Thr Gly Ile Asp Ile Asn
   365                 370                 375
   Leu Pro Gly Gly Gly Ala Leu Trp Ile Ile Gly Asp Val
           380                 385                 390
   Phe Leu Arg Lys Tyr Tyr Thr Val Tyr Asp His Gly Arg
                   395                 400
   Asp Ala Val Gly Phe Ala Leu Ala Lys
       405                 410
<210> 2
   <211> 412
   <212> PRT
   <213> *Trametes hirsuta*
<220>
   <221>
   <222> (1) ... (412)
   <223> mittels Sequenzierung des aus Stamm FCC 047 isolierten Enzyms bestimmte Aminosäuresequenz
<400> 2
   Met Ile Leu Ser Arg Phe Ala Pro Leu Ala Leu Leu Pro
   1               5                   10
   Phe Val Ala Ala Asp Gly Val His Lys Leu Lys Leu Thr
       15                  20                  25
   Lys Leu Pro Pro Ala Thr Ser Asn Pro Leu Leu Glu Ser
               30                  35
   Ala Tyr Leu Ala Glu Lys Tyr Gly Gly Gly Ser Gln Met
   40                  45                  50
   Pro Leu Ser Ala Gly Ile Gly Arg Asn Val Arg Val Ser
           55                  60                  65
   Arg Pro Thr Val Lys Asp Gly Glu Glu Leu Phe Trp Thr
                   70                  75
   Gln Asp Glu Phe Ser Thr Glu Gly Gly His Asn Val Pro
       80                  85                  90
   Leu Ser Asn Phe Met Asn Ala Gln Tyr Phe Ala Glu Ile
               95                  100
   Thr Leu Gly Thr Pro Pro Gln Ser Phe Lys Val Ile Leu
   105                 110                 115
   Asp Thr Gly Ser Ser Asn Leu Trp Val Pro Ser Thr Lys
           120                 125                 130
   Cys Thr Ser Ile Ala Cys Phe Leu His Ala Lys Tyr Asp
                   135                 140
   Ser Thr Ala Ser Ser Thr Tyr Lys Ala Asn Gly Ser Glu
       145                 150                 155
   Phe Ser Ile Gln Tyr Gly Ser Gly Ser Met Glu Gly Phe
               160                 165
   Val Ser Gln Asp Val Leu Thr Ile Gly Asp Ile Thr Ile
   170                 175                 180
   Lys Asn Gln Asp Phe Ala Glu Ala Thr Lys Glu Pro Gly
           185                 190                 195
   Leu Ala Phe Ala Phe Gly Lys Phe Asp Gly Ile Leu Gly
                   200                 205
   Leu Gly Tyr Asp Thr Ile Ser Val Asn His Ile Thr Pro
       210                 215                 220
   Pro Phe Tyr Gln Met Met Asn Gln Lys Leu Val Asp Ser
               225                 230
   Pro Val Phe Ser Phe Arg Leu Gly Ser Ser Glu Glu Asp
   235                 240                 245
   Gly Gly Glu Ala Ile Phe Gly Gly Val Asp Glu Thr Ala
           250                 255                 260
   Tyr Ser Gly Lys Ile Glu Tyr Val Pro Val Arg Arg Lys
                   265                 270
   Ala Tyr Trp Glu Val Glu Leu Glu Ser Ile Lys Leu Gly
       275                 280                 285
   Asp Asp Glu Leu Glu Leu Asp Asn Thr Gly Ala Ala Ile
               290                 295
   Asp Thr Gly Thr Ser Leu Ile Ala Leu Pro Ser Asp Leu
   300                 305                 310
   Ala Glu Met Leu Asn Ala Gln Ile Gly Ala Lys Lys Ser
           315                 320                 325
   Trp Asn Gly Gln Tyr Thr Val Asp Cys Ala Lys Val Pro
                   330                 335
   Thr Leu Pro Asp Leu Thr Phe Tyr Phe Ser Gly Lys Pro
       340                 345                 350
   Tyr Thr Leu Lys Gly Thr Asp Tyr Val Leu Glu Val Gln
               355                 360
   Gly Thr Cys Met Ser Ser Phe Thr Gly Ile Asp Ile Asn
   365                 370                 375
   Leu Pro Gly Gly Gly Ala Leu Trp Ile Ile Gly Asp Val
           380                 385                 390
   Phe Leu Arg Lys Tyr Tyr Thr Val Tyr Asp His Gly Arg
                   395                 400
   Asp Ala Val Gly Phe Ala Leu Ala Lys
       405                 410
<210> 3
   <211> 1237
   <212> DNA
   <213> *Trametes hirsuta*
<220>
   <221> CDS
   <222> (1) ... (1237)
   <223> für Seq.-ID Nr. 1 kodierende Sequenz
<400> 3
   atgatactct ccagattcgc ccccctcgcc ctgctcccct tcgtggccgc cgacggcgtc   60
   cacaagctga agctcaccaa gcttcctccc gcaacttcca acccgttgtt ggagagtgct  120
   tacctggctg agaagtatgg tggtggttcc cagatgcccc ttagcgcggg cattggccgc  180
   aacgtccgcg tgtcgcgccc gaccgtcaag gacggcgagg agctcttctg gactcaggac  240
   gagttttcga ccgagggcgg tcacaacgtt cccttgagta acttcatgaa cgctcagtac  300
   ttcgctgaaa tcaccttggg cactcccccg caatcgttca aggtcatcct ggacactggg  360
   tcgagcaacc tctgggttcc gagcaccaag tgtacctcca ttgcgtgctt cctacacgcc  420
   aagtatgact cgaccgcttc gtcgacatac aaggcgaacg gctccgagtt ctcgatccag  480
   tatggctctg gctccatgga gggcttcgtc tcgcaagatg tcttgacaat cggtgacatc  540
   accatcaaga accaagattt cgcagaggcc accaaggagc ccggcctcgc atttgccttt  600
   ggcaagtttg atggtatcct cggcctcggg tatgacacca tttccgtgaa ccacatcact  660
   cctcccttct accagatgat gaaccagaag ctcgtcgatt ctcctgtgtt ctctttccgc  720
   ctcggtagct cggaagagga cggtggtgaa gccatcttcg gaggagtcga tgagaccgcg  780
   tacagtggca agatcgaata cgtccctgtc aggaggaagg cgtactggga ggtggagctg  840
   gaatcgatca aactcggaga cgacgagctt gagctcgata acaccggcgc tgccatcgac  900
   actggaacct cgttgattgc tctcccctcc gatctggcgg agatgctcaa tgtgcaaatc  960
   ggtgccaaga agtcctggaa tggtcagtac accgtcgact gcgcgaaggt ccctaccctc 1020
   cccgacctca  ccttctactt  cagcggcaag  ccttacactc  tcaagggtac  cgactacgtc  1080
   ctcgaagttc agggaacttg catgtcctcg ttcaccggca tcgacatcaa tctgcccggc 1140
   ggtggtgctc tgtggatcat tggtgatgtc ttcctgcgca agtactacac tgtgtacgac 1200
   catggtcgcg atgccgttgg cttcgctctt gccaagt                          1237
<210> 4
   <211> 1237
   <212> DNA
   <213> *Trametes hirsuta*
<220>
   <221> CDS
   <222> (1) ... (1237)
   <223> für Seq.-ID Nr. 2 kodierende Sequenz
<400> 4
   atgatactct ccagattcgc ccccctcgcc ctgctcccct tcgtggccgc cgacggcgtc   60
   cacaagctga agctcaccaa gcttcctccc gcaacttcca acccgttgtt ggagagtgct  120
   tacctggctg agaagtatgg tggtggttcc cagatgcccc ttagcgcggg cattggccgc  180
   aacgtccgcg tgtcgcgccc gaccgtcaag gacggcgagg agctcttctg gactcaggac  240
   gagttttcga ccgagggcgg tcacaacgtt cccttgagta acttcatgaa cgctcagtac  300
   ttcgctgaaa tcaccttggg cactcccccg caatcgttca aggtcatcct ggacactggg  360
   tcgagcaacc tctgggttcc gagcaccaag tgtacctcca ttgcgtgctt cctacacgcc  420
   aagtatgact cgaccgcttc gtcgacatac aaggcgaacg gctccgagtt ctcgatccag  480
   tatggctctg gctccatgga gggcttcgtc tcgcaagatg tcttgacaat cggtgacatc  540
   accatcaaga accaagattt cgcagaggcc accaaggagc ccggcctcgc atttgccttt  600
   ggcaagtttg atggtatcct cggcctcggg tatgacacca tttccgtgaa ccacatcact  660
   cctcccttct accagatgat gaaccagaag ctcgtcgatt ctcctgtgtt ctctttccgc  720
   ctcggtagct cggaagagga cggtggtgaa gccatcttcg gaggagtcga tgagaccgcg  780
   tacagtggca agatcgaata cgtccctgtc aggaggaagg cgtactggga ggtggagctg  840
   gaatcgatca aactcggaga cgacgagctt gagctcgata acaccggcgc tgccatcgac  900
   actggaacct cgttgattgc tctcccctcc gatctggcgg agatgctcaa tgcgcaaatc  960
   ggtgccaaga agtcctggaa tggtcagtac accgtcgact gcgcgaaggt ccctaccctc 1020
   cccgacctca ccttctactt cagcggcaag ccttacactc tcaagggtac cgactacgtc 1080
   ctcgaagttc agggaacttg catgtcctcg ttcaccggca tcgacatcaa tctgcccggc 1140
   ggtggtgctc tgtggatcat tggtgatgtc ttcctgcgca agtactacac tgtgtacgac 1200
   catggtcgcg atgccgttgg cttcgctctt gccaagt                          1237